(19) Europäisches Patentamt
European Patent Office
Office européen des brevets

(11) Numéro de publication: **0 269 512 B1**

(12) # FASCICULE DE BREVET EUROPEEN

(45) Date de publication de fascicule du brevet: **11.09.91**

(51) Int. Cl.⁵: **C07D 501/34**, A61K 31/545

(21) Numéro de dépôt: **87402594.3**

(22) Date de dépôt: **18.11.87**

Le dossier contient des informations techniques présentées postérieurement au dépôt de la demande et ne figurant pas dans le présent fascicule.

(54) **Dérivés des céphalosporines à pharmacocinétique améliorée, procédé pour leur préparation et compositions pharmaceutiques les contenant.**

(30) Priorité: **20.11.86 FR 8616175**

(43) Date de publication de la demande: **01.06.88 Bulletin 88/22**

(45) Mention de la délivrance du brevet: **11.09.91 Bulletin 91/37**

(84) Etats contractants désignés: **AT BE CH DE ES FR GB GR IT LI LU NL SE**

(56) Documents cités:
**EP-A- 0 127 543**
**EP-A- 0 178 980**

(73) Titulaire: **SANOFI**
**40, Avenue George V**
**F-75008 Paris(FR)**

(72) Inventeur: **Olliero, Dominique**
**Hameau de l'Aiguelongue, Rue Jeanne Bourgeois n 12**
**F-34100 Montpellier(FR)**
Inventeur: **Labeeuw, Bernard**
**22, Rue Paul Eluard**
**F-34080 Montpellier(FR)**
Inventeur: **Roche, Gilles**
**8, Rue des Aires**
**F-34430 St. Jean De Vedas(FR)**
Inventeur: **Salhi, Ali**
**639, Rue de Valène**
**F-34980 Saint Gely Du Fesc(FR)**

(74) Mandataire: **Gillard, Marie-Louise et al**
**Cabinet Beau de Loménie 55, Rue d'Amsterdam**
**F-75008 Paris(FR)**

EP 0 269 512 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

La présente invention a pour objet de nouveaux dérivés des céphalosporines, un procédé pour leur préparation et des compositions pharmaceutiques les contenant en tant que principe actif.

Dans la demande de brevet français n° 84 14 878 publiée le 28/03/86 sous le numéro 2 570 702, la Demanderesse a décrit une famille de dérivés de céphalosporines possédant une activité large aussi bien sur les germes Gram négatif que les germes Gram positif.

Parmi ces dérivés figurent notamment des composés substitués en position 3 par un groupe :

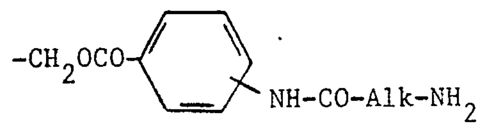

où Alk représente un groupe alkyle inférieur éventuellement substitué et où le substituant NH-CO-Alk-$NH_2$ est situé en position 3 ou 4.

Selon la présente invention, on a trouvé que de façon surprenante en modifiant légèrement la nature du substituant en position 3, on obtenait des composés qui conservaient la bonne activité des composés antérieurement décrits mais présentaient en plus des propriétés pharmacocinétiques très améliorées et notamment des concentrations plasmatiques très élevées persistant pendant un temps très important.

Une telle modification des paramètres pharmacocinétiques est importante dans la mesure où elle permet d'envisager la réduction de la posologie et du nombre de prises du produit pour obtenir un même effet thérapeutique.

Les composés selon l'invention, répondent à la formule générale :

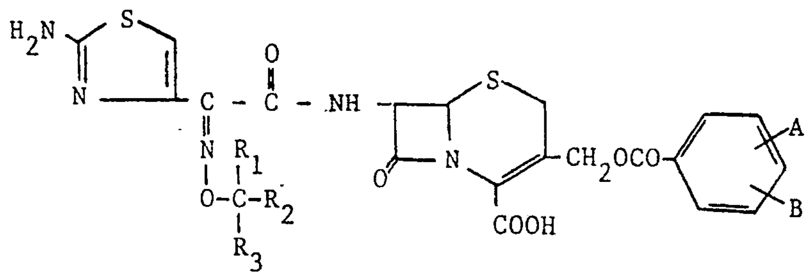

(I)

dans laquelle :

- $R_1$,$R_2$ et $R_3$ désignent chacun un atome d'hydrogène ou encore $R_1$ et $R_2$ désignent chacun un atome d'hydrogène ou un groupe méthyle et $R_3$ désigne un groupe carboxyle, ou encore $R_1$ et $R_2$ pris ensemble avec l'atome de carbone auquel ils sont liés forment un noyau cyclobutyle et $R_3$ désigne un groupe carboxyle,
- A et B sont différents et occupent les positions méta et para du noyau benzénique ;
  l'un représente un groupe OH et l'autre désigne soit :
  - un groupe

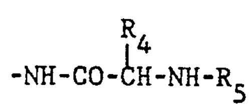

    où
    $R_4$ représente l'hydrogène, un groupe méthyle, un groupe hydroxyméthyle, un groupe $(CH_2)_n$-$NH_2$ où n est un entier compris entre 1 et 4 et $R_5$ désigne l'hydrogène ou lorsque $R_4$ représente l'hydrogène, $R_5$ peut aussi représenter un groupe alkyle inférieur, comportant de 1 à 4 atomes de carbone,
    soit
  - un groupe -NH-CO-$CH_2CH_2NH_2$

soit

- un groupe

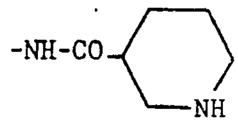

Avantageusement, le groupe hydroxyle (A ou B) est situé en position méta du noyau benzénique.

Par suite de la présence dans leur formule d'un groupe oxime, les composés (I) peuvent exister sous 2 formes isomères syn et anti. Les isomères syn, dont l'activité thérapeutique est supérieure, sont les composés préférés.

Lorsque l'un des substituants A ou B représente un groupe

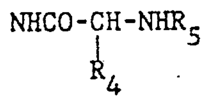

où $R_4$ est autre que l'hydrogène, l'atome de carbone portant $R_4$ est un carbone asymétrique. Par suite les composés (I) peuvent exister sous forme de diastéréoisomères (issus des formes D ou L de l'aminoacide) ou sous la forme du mélange des 2 diastéréoisomères (issu de la forme DL de l'aminoacide). L'ensemble de ces formes appartient à l'invention.

Avantageusement, les substituants A et B des composés selon l'invention représentent respectivement, le groupe hydroxyle en position méta et le groupe $-NHCOCH_2NH_2$ en position para.

Il est entendu que les composés (I) indiqués ci-dessous peuvent exister

- soit sous la forme indiquée dans la formule (I)
- soit sous la forme tautomère (I') :

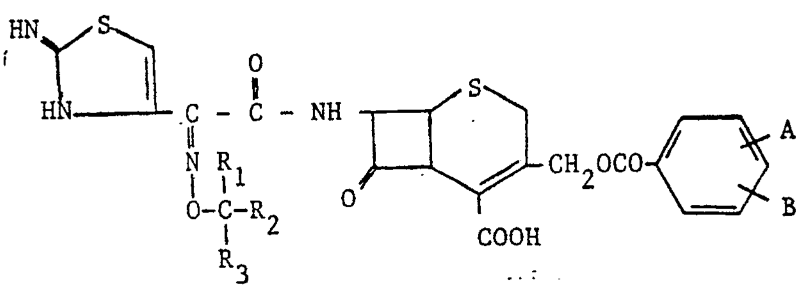

(I')

dans laquelle $R_1$, $R_2$, $R_3$, A et B sont tels que définis précédemment.

Les sels de composés de formule I (ou I') font partie intégrante de l'invention.

Il s'agit aussi bien de sels avec les acides pharmaceutiquement acceptables qui peuvent se former avec les fonctions aminées de la molécule que des sels alcalins, alcalinoterreux ou des sels d'aminoacides ou d'amines telles que la triéthylamine ou les éthanolamines qui sont susceptibles de se former avec le groupe carboxyle en position 4 du composé (I) ou lorsqu'il existe avec le groupe carboxyle présent dans le substituant de l'oxime ou encore avec les 2 groupes carboxyliques.

Il en est de même pour les esters facilement hydrolysables ou métaboliquement labiles dérivant de l'un ou de l'autre ou des 2 groupes carboxyliques éventuellement présents dans la molécule.

Parmi ces esters, on peut notamment mentionner :

les esters de phtalidyle

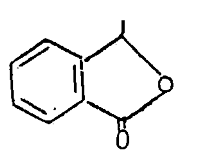

les esters d'acétoxy-1 éthyle

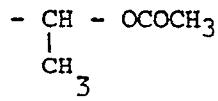

les esters d'éthoxycarbonyloxy-1 éthyle

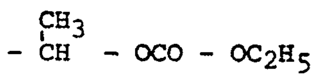

les esters de (méthyl-4 oxo-2 dioxolen-4yl-5) méthyle

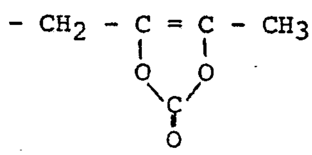

L'invention concerne également un procédé de préparation des composés de formule I (ou I') représenté par le schéma réactionnel suivant :

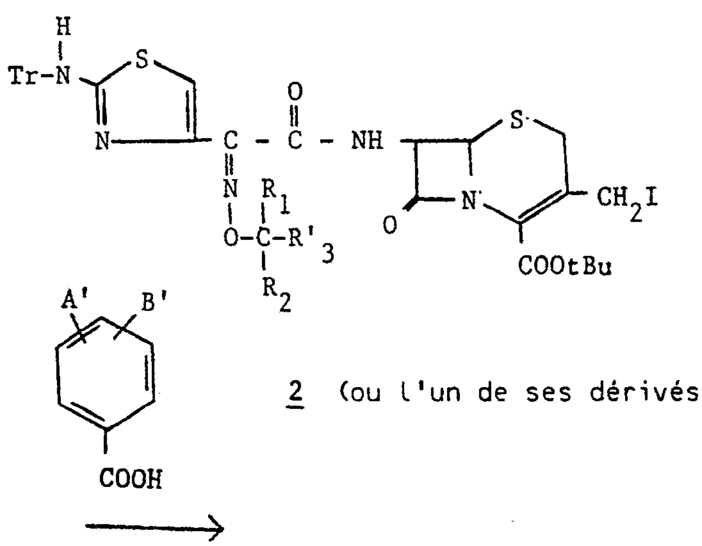

1

2 (ou l'un de ses dérivés réactifs)

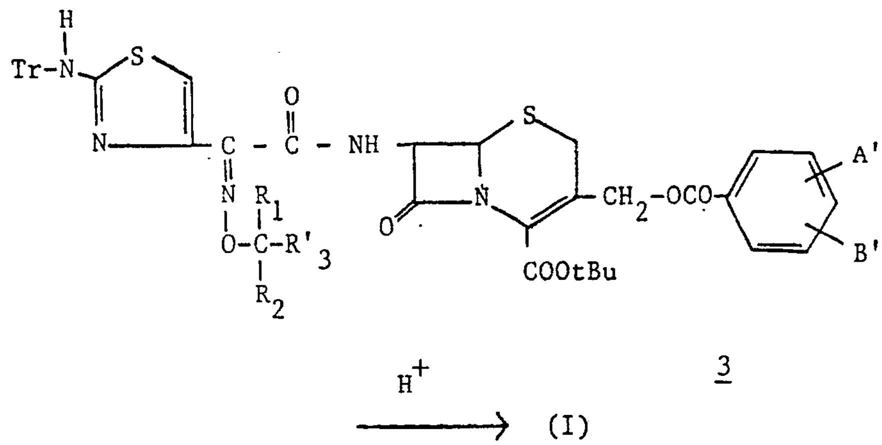

3

Dans ces formules, Tr représente un groupe protecteur de la fonction amine, de préférence le groupe trityle, tBu représente le groupe tertiobutyle et $R'_3$ désigne l'hydrogène ou un groupe ester facilement labile et de préférence un groupe COOtBu.

A'et B' qui occupent les positions méta et para du noyau benzénique sont différents et représentent l'un un groupe OH et l'autre un groupe dérivant du groupe B par blocage du ou des groupes aminés qui y sont présents par un groupe labile.

Enfin $R_1$, $R_2$, $R_3$, A et B ont les significations précédemment définies.

Sur le composé iodé 1, on fait agir l'acide 2 dans lequel la ou les fonctions amines ont été préalablement protégées, selon une méthode connue, par un groupement tel que le tertiobutoxycarbonyle ou le trichloroéthoxycarbonyle.

D'une façon générale la réaction a lieu en solution dans un solvant convenable de préférence le diméthylformamide en présence de bicarbonate de potassium ou d'une amine tertiaire peu nucléophile comme la diisopropyléthylamine.

La réaction est effectuée à température peu élevée de 0 à 20 °C.

A partir du composé protégé 3 ainsi obtenu, on prépare les composés (I) par élimination des groupes protecteurs portés par les fonctions amines et carboxyliques selon un procédé connu, en particulier par hydrolyse en milieu acide en utilisant par exemple l'acide trifluoroacétique ou un mélange acide formique-acide chlorhydrique.

Dans ces conditions, le composé (I) est isolé directement sous forme de sel du groupement aminé avec l'acide fort utilisé pour la déprotection, c'est à dire sous forme de trifluoracétate ou de chlorhydrate.

Eventuellement, on peut transformer ces sels en d'autres sels d'acide fort, par passage d'une solution de chlorhydrate ou de trifluoracétate sur une résine échangeuse d'ions basique sous forme de sel d'acide faible (formiate ou acétate par exemple).

A la solution ainsi obtenue on ajoute l'acide fort dont on veut obtenir le sel et on isole le sel ainsi obtenu par exemple par lyophilisation.

Les dérivés iodés 1 utilisés comme produits de départ sont connus ou peuvent être préparés selon un procédé connu et notamment comme indiqué dans la demande de brevet allemand n° 3 311 300.

Les amino acides protégés 2 sont préparés à partir des hydroxyamino acides correspondants selon le schéma :

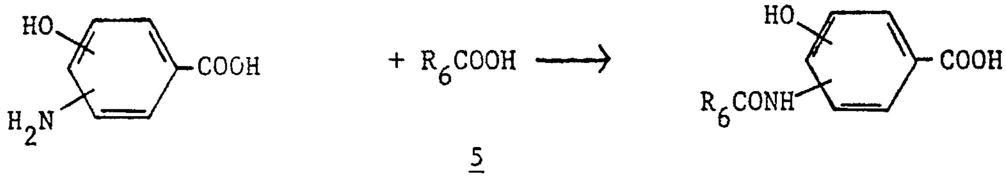

+ $R_6COOH$

5

4 2

où $R_6$ représente les groupes correspondant à

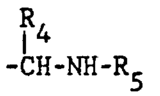 , $-(CH_2)_2NH_2$ ou 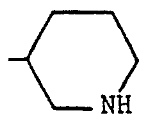

dans lesquels la ou les fonctions amines ont été protégées, et où $R_4$ et $R_5$ sont tels que définis précédemment.

La réaction d'amidification s'effectue en général non pas avec l'acide $R_6COOH$ mais avec un ester activé de celui-ci tel que l'ester de N-hydroxysuccimide ou l'ester de N-hydroxy bicyclo [2.2.1] heptène-5 dicarboxylique acide-2,3 imide.

La réaction est effectuée en solution dans le diméthylformamide par chauffage entre 30 et 80° C.

Les sels et esters d'acide carboxylique des composés (I) de l'invention s'obtiennent à partir des composés (I) par des réactions connues en elles-mêmes.

Ainsi, les sels minéraux sont obtenus par action sur les composés (I) d'une base minérale telle que la soude ou la potasse ou le bicarbonate de sodium en quantité équimoléculaire ; la réaction de salification est réalisée dans un solvant tel que l'eau ou l'éthanol et le sel obtenu est isolé par évaporation de la solution.

Les sels de bases organiques sont obtenus par action, sur une solution de l'acide I, dans un solvant ou un mélange de solvants convenables, d'une quantité équimoléculaire de la base organique. Le sel est isolé par précipitation avec l'éther. Les esters sont obtenus par les procédés connus d'estérification ; par exemple, on utilisera avantageusement l'action d'un dérivé halogéné sur un sel tel que le sel de sodium de l'acide ; on réalisera de préférence la réaction dans un solvant capable de dissoudre le dérivé acide de départ, par exemple, dans le diméthylformamide.

Les isomères de forme syn et anti s'obtiennent par un choix convenable des réactifs.

Les exemples suivants permettront de mieux comprendre la portée de l'invention sans toutefois la limiter.

Ainsi qu'il est habituel dans cette famille de composés, les produits selon l'invention ne présentent pas de point de fusion net, mais seulement des points de décomposition ne permettant pas de les caractériser.

Les produits seront donc caractérisés par leur spectre de résonance magnétique nucléaire. Sauf indication contraire, ils sont enregistrés à 250 MHz, l'étalon interne étant l'hexaméthyldisiloxane.

Les spectres sont enregistrés dans le diméthylsulfoxyde deutérié : 10 mg dans 0,5 ml.

Les déplacements chimiques sont mesurés à ± 0,01 ppm et les constantes de couplage à ± 0,5 Hz.

Les abréviations suivantes seront utilisées :

- S : singulet
- D : doublet
- D de D : doublet de doublet
- S. e. : singulet élargi
- M : multiplet
- Q : quadruplet
- T : triplet
- AB : système AB
- J : représente la constante de couplage.

De plus, les microanalyses élémentaires ont été effectuées dans chaque cas et sont en accord avec les formules indiquées.

EXEMPLE 1

Bis trifluoracétate de l'acide [ (amino-2 thiazolyl-4)-2 (carboxy-2 propyl-2 oxyimino)-2 acétamido ]-7 [ [ (amino-2 acétyl) amino-4 hydroxy-3 benzoyl]-oxyméthyl]-3 céphème-3 carboxylique-4 isomère syn (SR 43753)

(I) $R_1 = R_2 = -CH_3$ $R_3$ = COOH A = -OH (3) B = $-NH-CO-CH_2-NH_2$ (4)

A) Acide hydroxy-3 [(tertiobutoxy-carbonylamino-2 acétyl) amino]-4 benzoïque.

On chauffe à 80° C la solution de 9,75 g d'acide amino-4 hydroxy-3 benzoïque et 9,75 ml de triéthylamine dans 90 ml de diméthylformamide. On ajoute 20 g d'ester de N-hydroxysuccinimide de la N-tertiobutoxycarbonylglycine et maintient le mélange réactionnel pendant 4 heures à 80° C.

On évapore le solvant sous vide et reprend le résidu dans le minimum d'eau et on verse dans 2 litres de tampon sulfate pH2.

On essore le solide qui se sépare, lave avec de l'eau et dissout dans 1,2 litre d'acétate d'éthyle. On lave la solution organique 3 fois avec 500 ml d'eau puis on la sèche sur sulfate de magnésium. On évapore le solvant à siccité et reprend le résidu solide dans 300 ml de dichlorométhane sous forte agitation.

On essore les cristaux, lave au dichlorométhane et sèche à 100° C. On obtient 16,5 g du produit attendu F>250° C.

SPECTRE RMN enregistré à 60 MHz en solution dans le diméthylsulfoxyde deutérié.

1H à 9,13 ppm (S, Ar NH CO) -1H à 8;20 ppm (D, J = 8 Hz, H aromatique méta COOH) -2H à 7,47 ppm (M, H aromatiques ortho COOH) -1H à 7,38 ppm (T, J = 7 Hz, -NH -Boc) 2H à 3,77 ppm (D, J = 7 Hz, -C(= O)-$CH_2$-N )-9H à 1,30 ppm (S, -C $(CH_3)_3$).

B) [(Tritylamino-2 thiazolyl-4)-2 (tertiobutoxycarbonyl-2 propyl-2 oxyimino)-2 acétamido]-7 [[-(tertiobutoxycarbonylamino-2 acétyl) amino-4 hydroxy-3 benzoyl] oxyméthyl]-3 céphème-3 carboxylate de tertiobutyle-4 isomère syn

On dissout 15 g de l'acide protégé préparé en A et 10,9 ml de diisopropyléthylamine dans 100 ml de diméthylformamide anhydre.

On refroidit la solution à 4° C et ajoute 36 g de [ (tritylamino-2 thiazolyl-4)-2(tertiobutoxycarbonyl-2 propyl-2 oxyimino)-2 acétamido ]-7 iodométhyl-3 céphème-3 carboxylate de tertiobutyle-4 isomère syn. On laisse sous agitation à 4° C pendant 5 heures puis verse le mélange réactionnel sur une solution glacée de tampon sulfate pH2. On agite vigoureusement pendant 10 minutes puis on essore le solide et lave avec de l'eau. On reprend le solide dans l'acétate d'éthyle et lave la solution organique avec du tampon sulfate pH2, puis avec de l'eau, avec une solution saturée de bicarbonate de sodium et enfin avec de l'eau. On sèche la solution sur sulfate de magnésium puis évapore le solvant à siccité.

On chromatographie le produit obtenu sur colonne de silice H. En éluant avec un mélange dichlorométhane-acétate d'éthyle 90-10 (vol/vol), on obtient 25 g du produit attendu utilisé tel quel pour l'étape suivante.

SPECTRE DE RMN

1H à 10,5 ppm (s. e., OH )-1H à 9,35 ppm (D, J = 9Hz, -C(= O)-NH -)-1H à 9,10 ppm (S, ArNH CO-)-1H à 8,80 ppm (S, NH -Trityle)-1H à 8,15 ppm (D, J = 8 Hz, H aromatique méta CO)-18H à 7,25 ppm (M, H aromatiques Trityle, H aromatiques ortho CO et-NH -Boc)-1H à 6,64 ppm (S, H thiazole)-1H à 5,73 ppm (D de D $J_1$ = 9Hz $J_2$ = 4Hz, H 7)-2H à 5,10 ppm (M, $H_6$ et $CH_2OC$(= 0)-)-1H à 4,81 ppm (D, J = 13 Hz, $CH_2OC$ = O)-2H à 3,72 ppm (D, J = 7Hz, CO-$CH_2$-N )-2H à 3,57 ppm (AB, JAB = 17Hz, $CH_2$ S)-33H à 1,30 ppm (3S, H-Boc et -C-$(CH_3)_2$).

C) SR 43753

A un mélange de 250 ml d'acide trifluoracétique et 25 ml d'anisole, on ajoute par petites fractions 25 g du produit protégé obtenu en B.

L'addition terminée, on laisse 1 heure à 25° C puis on évapore à siccité sous vide. On ajoute au résidu

500 ml d'éther anhydre et laisse 15 minutes sous forte agitation. On essore le solide et lave 3 fois avec de l'éther anhydre puis sèche sous vide.

On obtient 18 g du produit attendu.

SPECTRE DE RMN

1H à 10,60 ppm (s. e., OH )-1H à 9,91 ppm (S, ArNH CO)-1H à 9,42 ppm (D, J = 9Hz, -CO-NH -)-4H à 8,10 ppm (M, $NH_3^+$ glycine et H aromatique méta C = 0)-4H à 7,40 ppm (M, H aromatiques ortho CO et $NH_2$thiazole)-1H à 6,71 ppm (S, H thiazole)-1H à 5,82 ppm (D de D, $J_1$ = 9Hz $J_2$ = 4Hz, $H_7$)-2H à 5,20 ppm (M, $H_6$5et $CH_2OCO$)-1H à 4,84 ppm (D, J = 13 Hz, $CH_2OCO$)-2H à 3,85 ppm (M, CO-$CH_2$-N)-2H à 3,64 ppm (AB, $J_{AB}$ = 17Hz, $CH_2S$)-6H à 1,40 ppm (2S, -N-O-C-$(CH_3)_2$)

EXEMPLES 2 A 15

A) En opérant comme dans l'exemple 1-A mais en faisant varier les produits mis en réaction, on obtient de la même façon les acides protégés réunis dans le tableau 1.

TABLEAU 1

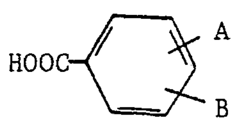

| A | B | Spectre de RMN N° ou point de fusion F : |
|---|---|---|
| $-NHCOCH_2NH-Boc$ | -OH | 1 |
| -OH | $-NHCOCH_2CH_2NH-Boc$ | 2 |
| -OH | -NH-CO-CH-NH-Boc<br>\|<br>$CH_3$ | 3 |
| -OH | -NHCO-CH-NHBoc<br>\|<br>$CH_2OH$ | 4 |
| -OH | $-NH-COCH_2$-N-Boc<br>\|<br>$CH_3$ | 5 |
| -OH | -NH-CO 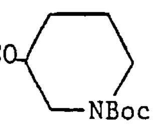 | F : 210-2°C |
| -OH | $-NH-CO-CH-(CH_2)_3NH-Boc$<br>\|<br>NH-Boc | 6 |
| -OH | $-NH-CO-CH-(CH_2)_2NHBoc$<br>\|<br>NH-Boc | 7 |

SPECTRE DE RMN N°1 (60 MHz, DMSO)

1H à 12,60 ppm (s. e., COOH )-1H à 10,40 ppm (s. e., OH )-1H à 9,05 ppm (S, Ar NH -)-1H à 8,60 ppm (D, J = 3Hz, H aromatique ortho NH)-1H à 7,57 ppm (D de D, $J_1$ = 8Hz $J_2$ = 3Hz, H aromatique para NH)-1H à 7,20 ppm (T, J = 7Hz, -NH Boc) 1H à 6,90 ppm (D, J = 8Hz, H aromatique méta NH)-2H à 3,72 ppm (D, J = 7Hz, -C(= O)-$CH_2$-N<)-9H à 1,35 ppm (S, Boc).

SPECTRE DE RMN N°2

1H à 12,50 ppm (s. e., COOH )-1H à 10,43 ppm (s. e., OH ) 1H à 9,24 ppm (S, Ar-NH -)- 1H à 8,00 ppm (D, J = 8Hz, H aromatique ortho NH)- 1H à 7,40 ppm (S, H aromatique ortho OH)- 1H à 7,34 ppm (D, J = 8Hz, H aromatique para OH)- 1H à 6,81 ppm (T, J = 7Hz, NH -Boc)- 2H à 3,18 ppm (Q, J = 7Hz, -$CH_2$NH-Boc)- 2H à 2,52 ppm (T, J = 7Hz, $CH_2CH_2$ NHBoc) 9H à 1,33 ppm (S, Boc).

SPECTRE DE RMN N°3 (60 MHz, DMSO).

1H à 12,40 ppm (s. e., COOH)- 1H à 10,40 ppm (s. e., OH) 1H à 9,10 ppm (S, Ar NH)-1H à 8,10 ppm (D, J = 8Hz, H aromatique ortho NH)- 3H à 7,40 ppm (M, -NH-Boc + 2H aromatiques)- 1H à 4,12 ppm

(M, -CO-CH-N )-
CH$_3$

9H à 1,34 ppm (S, Boc)- 3H à 1,27 ppm

(D, J = 7Hz, -CH-N )
CH$_3$

SPECTRE DE RMN N°4

1H à 12,60 ppm (s. e., COOH )- 1H à 10,45 ppm (s. e., Ar-OH ) 1H à 9,22 ppm (S, Ar NH CO)- 1H à 8,18 ppm (D, J = 8Hz, H aromatique ortho NH)- 2H à 7,40 ppm (H aromatique méta NH)- 1H à 7,15 ppm (D, J = 7Hz, NH Boc)- 1H à 5,06 ppm (s. e., $CH_2OH$ )- 1H à 4,17 ppm

(M, COCH-N)
CH$_2$OH

2H à 3,57 ppm

(M, CO-CH-N)-
CH$_2$OH

9H à 1,34 ppm (S, Boc)

SPECTRE DE RMN N°5 (60 MHz, DMSO)

1H à 12,6 ppm (s. e., COOH )- 1H à 10,47 ppm (s. e., OH ) 1H à 9,21 ppm (S, Ar NH -CO)- 1H à 8,10 ppm (D, J = 8Hz, H aromatique para OH)- 2H à 7,43 ppm (M, H aromatiques)- 2H à 4,00 ppm (S, $COCH_2$ N)- 3H à 3,80 ppm (>N-$CH_3$)- 9H à 1,32 ppm (S, Boc)

SPECTRE DE RMN N°6

1H à 12,66 ppm (s. e., COOH )- 1H à 10,44 ppm (s. e.,OH )- 1H à 9,11 ppm (S, Ar NH CO)- 1H à 8,12 ppm (D, J = 8Hz, H aromatique ortho NH)- 3H à 7,40 ppm

(M, H aromatiques + -CH-CH$_2$)-
NHBoc

1H à 6,75 ppm (T, J = 7Hz, $CH_2NHBoc$)- 1H à 4,08 ppm

(M, -CH-CH$_2$-)-
N

2H à 2,81 ppm (M, $CH_2$-N<)- 4H entre 1,4 et 1,7 ppm (M, $CH_2$-$CH_2$)- 18H à 1,33 ppm (2S, Boc)-

SPECTRE DE RMN N°7

1H à 12,55 ppm (s. e., COOH)- 1H à 10,47 ppm (s. e., OH)- 1H à 9.15 ppm (S, Ar NHCO)- 1H à 8,13 ppm (D, J = 8Hz, H aromatique ortho NH)- 3H à 7,40 ppm

(M, H aromatiques + $-CH-CH_2$)-
|
NHBoc

1H à 6,78 ppm (T, J = 7Hz, $CH_2$NHBoc)- 1H à 4,13 ppm

(M, $CH-CH_2$)-
|
N

2H à 2,98 ppm (M, $-CH_2$NHBoc)- 1H à 1,92 ppm et 1H à 1,70 ppm

(M, $-CH-CH_2$)-
|
N
/\

18H à 1,33 ppm (2S, Boc)-

B) A partir de ces acides protégés et par action sur différents composés iodés selon l'exemple 1-B, on obtient les céphalosporines protégées correspondantes qui déprotégées selon la méthode de l'exemple 1-C conduisent aux différents composés (I) isolés comme trifluoracétates sous forme syn réunis dans le tableau 2.

TABLEAU 2

| N° d'exemple | N° de code SR | $R_1$ | $R_2$ | $R_3$ | 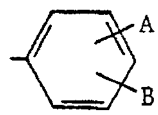 | RMN |
|---|---|---|---|---|---|---|
| 2 | 43851 | H | H | H | 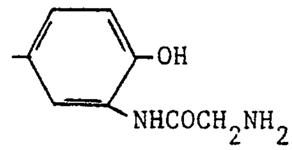 | 8 |
| 3 | 43852 | H | H | H | 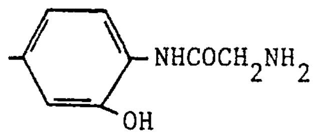 | 9 |
| 4 | 43853 | H | H | H | 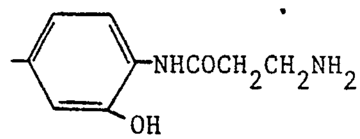 | 10 |
| 5 | 43903 | $CH_3$ | $CH_3$ | COOH | 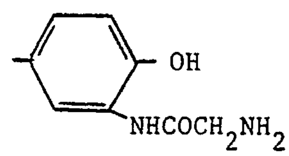 | 11 |
| 6 | 43904 | $CH_3$ | $CH_3$ | COOH | 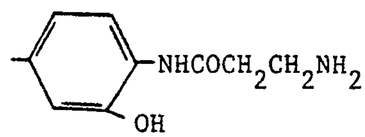 | 12 |
| 7 | 43955 | $CH_3$ | $CH_3$ | COOH | 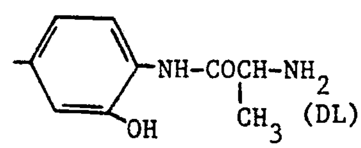 (DL) | 13 |

| | | | | | | |
|---|---|---|---|---|---|---|
| 8 | 43983 | H | H | H | 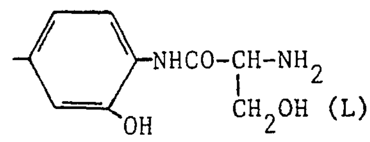 | 14 |
| 9 | 43984 | $CH_3$ | $CH_3$ | COOH | " " | 15 |
| 10 | 43985 | $CH_3$ | $CH_3$ | COOH | 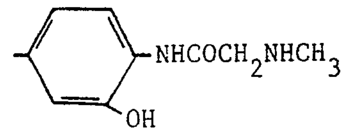 | 16 |
| 11 | 44049 | $CH_3$ | $CH_3$ | COOH | 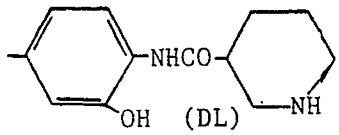 | 17 |
| 12 | 44128 | $CH_3$ | $CH_3$ | COOH | 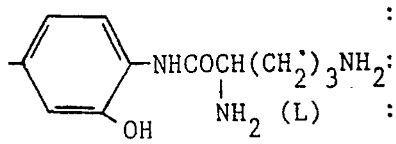 | 18 |
| 13 | 44130 | $CH_3$ | $CH_3$ | COOH | 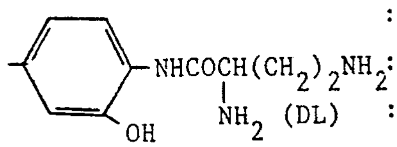 | 19 |
| 14 | 44218 | H | H | H | " " | 20 |
| 15 | 44219 | H | H | H | 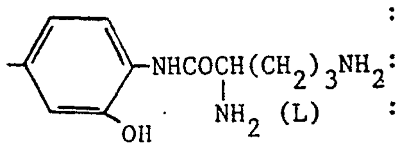 | 21 |

SPECTRE DE RMN N°8

1H à 11,13 ppm (s. e. OH )- 1H à 9,80 ppm (S, Ar NH CO)- 1H à 9,57 ppm (D, J = 9Hz, CONH )- 1H à 8,52 ppm (S, H aromatique ortho NH)- 3H à 8,02 ppm (s. e.,$CH_2$-$N^+_3$)- 1H à 7,61 ppm (D, J = 8Hz, H aromatique para NH)- 2H à 7,25 ppm (s. e., $NH_2$ thiazole)-1H à 6,98 ppm (D, J = 8Hz, H aromatique méta NH)- 1H à 6,70 ppm (S, H thiazole)- 1H à 5,77 ppm (D de D, $J_1$ = 9Hz $J_2$ = 4Hz, $H_7$)-1H à 5,20 ppm (D, J = 13Hz, $CH_2OCO$)- 1H à 5,16 ppm (D, J = 4Hz, $H_6$ )- 1H à 4,85 ppm (D, J = 13Hz, $CH_2OCO$)- 3H à 3,77 ppm (S, $NOCH_3$)-2H à 3,58 ppm (AB, $J_{AB}$ = 17Hz, $CH_2S$)-

SPECTRE DE RMN N°9

1H à 10,57 ppm (s. e. OH)- 1H à 9,90 ppm (S, Ar NHCO)- 1H à 9,61 ppm (D, J = 9Hz, CONH)- 4H à 8,10 ppm (M, H aromatique ortho NH et $CH_2N^+_3$)- 4H à 7,40 ppm (H aromatiques méta NH et $NH_2$ thiazole)-1H à 6,72 ppm (S, H thiazole)- 1H à 5,81 ppm (D de D, $J_1$ = 9Hz $J_2$ = 4Hz, $H_7$)- 2H à 5,10 ppm (M, $H_6$ et $CH_2OCO$)- 1H à 4,90 ppm (D, J - 13Hz, $CH_2OCO$)- 2H à 3,85 ppm (M, $CH_2NH_2$)- 3H à 3,77 ppm (S, N-$OCH_3$)- 2H à 3,65 ppm (AB, $J_{AB}$ = 17Hz, $CH_2S$)-

SPECTRE DE RMN N°10

1H à 10,47 ppm (S, OH)- 2H à 9,54 ppm (M, CONH et Ar NHCO)- 1H à 8,07 ppm (D, J = 8Hz, H aromatiques ortho NH)- 3H à 7,80 ppm (s. e. $CH_2N^+_3$)- 2H à 7,45 ppm (M, H aromatiques méta NH)- 2H à 7,20 ppm (s. e., $NH_2$ thiazole)- 1H à 6,70 ppm (S, H thiazole)- 1H à 5,78 ppm (D de D, $J_1$ = 9Hz $J_2$ = 4Hz, $H_7$)- 2H à 5,13 ppm (M, $H_6$ et $CH_2OCO$)- 1H à 4,87 ppm (D, J = 13Hz, $CH_2OCO$)-3H à 3,80 ppm (S, N-$OCH_3$)- 2H à 3,60 ppm (AB, $J_{AB}$ = 17Hz, $CH_2S$)- 2H à 3,05 ppm (M, $CH_2NH^+_3$)- 2H à 2,80 ppm (M, $CH_2CH_2NH_3$)-

SPECTRE DE RMN N°11

1 à 11,20 ppm (s. e., OH)- 1H à 9,80 ppm (S, Ar NHCO)- 1H à 9,56 ppm (D, J = 9Hz, CONH)- 1H à 8,52 ppm (S, H aromatique ortho NH)- 3H à 8,06 ppm (s. e., $CH_2NH_3^+$ )- 1H à 7,60 ppm (D, J = 8Hz, H aromatique para NH)- 2H à 7,30 ppm (s. e., $NH_2$ thiazole)-1H à 6,98 ppm (D, J = 8Hz, H aromatique méta NH)- 1H à 6,71 ppm (S, H thiazole)- 1H à 5,82 ppm (D de D, $J_1$ = 9Hz $J_2$ = 4Hz, $H_7$)- 2H à 5,15 ppm (M, $H_6$ et $CH_2OCO$) 1H à 4,81 ppm (D, J = 13Hz, $CH_2OCO$)- 2H à 3,84 ppm (M, $CH_2NH_3^+$ )- 2H à 3,60 ppm (AB, $J_{AB}$ = 17Hz, $CH_2S$)- 6H à 1,35 ppm (2S, -C-$(CH_3)_2$)

SPECTRE DE RMN N°12

1H à 10,48 ppm (s. e., OH)- 1H à 9,54 ppm (S, Ar NHCO)- 1H à 9,45 ppm (D, J = 9Hz, CONH)- 1H à 8,10 ppm (D, J = 8Hz, H aromatique ortho NH)- 3H à 7,70 ppm (s. e.,$CH_2NH_3^+$ )- 4H à 7,40 ppm (M, $NH_2$ thiazole, H aromatiques méta NH)- 1H à 6,70 ppm (S, H thiazole)-1H à 5,80 ppm (D de D, $J_1$ = 9Hz $J_2$ = 4Hz, $H_7$)- 2H à 5,15 ppm (M, $H_6$ et $CH_2OCO$)- 1H à 4,82 ppm (D, J = 13Hz, $CH_2OCO$)- 2H à 3,62 ppm (AB, $J_{AB}$ = 17Hz, $CH_2S$)- 2H à 3,05 ppm (M, $CH_2NH_3^+$ )- 2H à 2,80 ppm (M, $CH_2CH_2NH_3^+$ )- 6H à 1,34 ppm (2S, -C-$(CH_3)_2$ )

SPECTRE DE RMN N°13

1H à 10,60 ppm (s. e., OH)- 1H à 9,95 ppm (S, Ar NHCO)- 1H à 9,47 ppm (D, J = 9Hz, CONH)- 4H à 8,20 ppm (M,

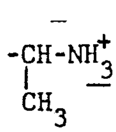

et H aromatiques ortho NH)- 4H à 7,40 ppm (M, $NH_2$ thiazole et H aromatiques méta NH)- 1H à 6,71 ppm (S, H thiazole)- 1H à 5,88 ppm (D de D, $J_1$ = 9Hz $J_2$ = 4Hz, $H_7$)- 2H à 5,20 ppm (M, $H_6$ et $CH_2OCO$)-1H à 4,84 ppm (D, J = 13Hz, $CH_2OCO$)- 1H à 4,20 ppm

(M, 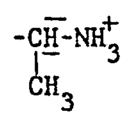 )-

2H à 3,62 ppm (AB, $J_{AB}$ = 17Hz, $CH_2S$)- 9H à 1,35 ppm (M, -C-$(CH_3)_2$ et

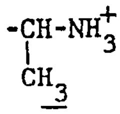 )

SPECTRE DE RMN N°14

1H à 10,62 ppm (s. e., OH)- 1H à 9,83 ppm (S, Ar NHCO)- 1H à 9,59 ppm (D, J = 9Hz, CONH)- 4H à 8,30 ppm (M,

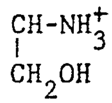

et H aromatique ortho NH)- 2H à 7,43 ppm(M, H aromatiques méta NH)- 2H à 7,25 ppm (s. e., $NH_2$ thiazole)- 1H à 6,69 ppm (S, H thiazole)- 1H à 5,79 ppm (D de D, $J_1$ = 9Hz $J_2$ = 4Hz, $H_7$)- 2H à 5,15 ppm (M, $H_6$ et $CH_2OCO$)-1H à 4,86 ppm (D, J = 13Hz, $CH_2OCO$)- 1H à 4,24 ppm

(M, 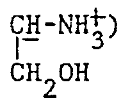 )-

3H à 3,81 ppm (S, $NOCH_3$)- 2H à 3,76 ppm

(D, J = 6Hz, 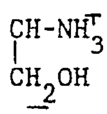 )-

2H à 3,63 ppm (AB, $J_{AB}$ = 17Hz, $CH_2S$)-

SPECTRE DE RMN N° 15

1H à 10,60 ppm (s. e., OH)- 1H à 9,90 ppm (S, Ar NHCO)- 1H à 9,42 ppm (D, J = 9Hz, CONH)- 4H à 8,20 ppm (M,

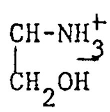

et H aromatique ortho NH)- 2H à 7,45 ppm (M, H aromatiques méta NH)-2H à 7,30 ppm (s. e., $NH_2$ thiazole)- 1H à 6,69 ppm (S, H thiazole)-1H à 5,84 ppm (D de D, $J_1$ = 9Hz $J_2$ = 4Hz, $H_7$)- 2H à 5,17 ppm (M, $H_6$ et $CH_2OCO$)- 1H à 4,84 ppm (D, J = 13Hz, $CH_2OCO$)-1H à 4,25 ppm

(M, 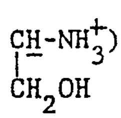 )-

2H à 3,75 ppm

(D, J = 6Hz, 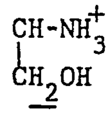)-

2H à 3,68 ppm (AB, $J_{AB}$ = 17Hz, $CH_2S$)- 6H à 1,35 ppm (S, $C(CH_3)_2$)-

SPECTRE DE RMN N° 16

1H à 10,57 ppm (s. e., OH)- 1H à 9,98 ppm (S, Ar NHCO)- 1H à 9,40 ppm (D, J = 9Hz, CONH)- 2H à 8,80 ppm (s. e., $CH_2NH_2^+$ -$CH_3$)-1H à 8,08 ppm (D, J = 8Hz, H aromatique ortho NH)- 2H à 7,45 ppm (M, H aromatiques méta NH)- 2H à 7,25 ppm (s. e., $NH_2$ thiazole)-1H à 6,68 ppm (S, H thiazole)- 1H à 5,82 ppm (D de D, $J_1$ = 9Hz $J_2$ = 4Hz, $H_7$)- 2H à 5,18 ppm (M, $H_6$ et $CH_2OCO$)- 1H à 4,83 ppm (D, J = 13Hz, $CH_2OCO$)- 2H à 4,00 ppm (M, $CH_2NH_2^+$ $CH_3$)- 2H à 3,61 ppm (AB, $J_{AB}$ = 17Hz, $CH_2S$)- 3H à 2,57 ppm (s. e., $CH_2NH_2^+$ -$CH_3$)-6H à 1,37 ppm (2S, $C(CH_3)_2$)-

SPECTRE DE RMN N° 17

1H à 10,42 ppm (s. e., OH)- 1H à 9,57 ppm (S, Ar NHCO)- 1H à 9,43 ppm (D, J = 9Hz, CONH)- 2H à 8,50 ppm

(s. e., 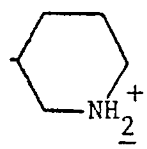 )-

1H à 8,05 ppm (D, J = 8Hz, H aromatique ortho NH)- 4H à 7,40 ppm (M, $NH_2$ thiazole et H aromatiques méta NH)- 1H à 6,69 ppm (S, H thiazole) 1H à 5,84 ppm (D de D, $J_1$ = 9Hz $J_2$ = 4Hz, $H_7$)- 2H à 5,20 ppm (M, $H_6$ et $CH_2OCO$)- 1H à 4,82 ppm (D, J = 13Hz, $CH_2OCO$)- 2H à 3,66 ppm (AB, $J_{AB}$= 17Hz, $CH_2S$)- 5H entre 2,5 et 3,5 ppm

(M, CO-CH et $CH_2N^+$)-

4H entre 1,45 et 2,05 ppm ($CH_2$ du cycle méta et para de $N^+$)-6H à 1,40 ppm (2S, $C(CH_3)_2$)-

SPECTRE DE RMN N°18

1H à 10,80 ppm (s. e., OH)- 1H à 10,00 ppm (S, Ar NHCO)- 1H à 9,44 ppm (D, J = 9Hz, CONH)- 3H à 8,20 ppm (s. e., $NH_3^+$ )- 1H à 8,05 ppm (D, J = 8Hz, H aromatique ortho NH)- 3H à 7,70 ppm (s.e., $NH_3^+$ )- 4H à 7,30 ppm ($NH_2$ thiazole et H aromatiques méta NH)- 1H à 6,66 ppm (S, H thiazole)- 1H à 5,82 ppm (D de D, $J_1$ = 9Hz $J_2$ = 4Hz, $H_7$)- 2H à 5,20 ppm (M, $H_6$ et $CH_2OCO$)- 1H à 4,84 ppm (D, J = 13Hz, $CH_2OCO$)- 1H à 4,42 ppm

(M, 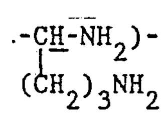)-

2H à 3,61 ppm (AB, $J_{AB}$ = 17Hz, $CH_2S$)- 2H à 2,83 ppm

(M, 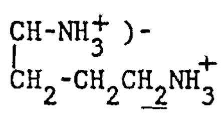 )-

2H à 1,80 ppm

(M, $-CH-NH_3^+)-$ 2H à 1,60 ppm
$\quad | $
$\quad CH_2\underline{CH_2}CH_2NH_3^+$
(2S, $C(\underline{CH_3})_2)-$

(M, $-CH-NH_3^+)-$ 6H à 1,39 ppm
$\quad | $
$\quad \underline{CH_2}CH_2CH_2NH_3^+$

SPECTRE DE RMN N°19

1H à 10,72 ppm (s. e., OH)- 1H à 10,00 ppm (S, Ar NHCO)- 1H à 9,36 ppm (D, J = 9Hz, CONH)- 3H à 8,40 ppm (s.e., $NH_3^+$ )- 1H à 8,11 ppm (D, J = 8Hz, H aromatique ortho NH)- 3H à 7,90 ppm (s. e., $NH_3^+$ )- 4H à 7,30 ppm (M, H aromatiques méta NH et $NH_2$ thiazole)-1H à 6,66 ppm (S, H thiazole)- 1H à 5,81 ppm (D de D, $J_1$ = 9Hz $J_2$ = 4Hz, $H_7$)- 2H à 5,15 ppm (M, $H_6$ et $CH_2OCO$)- 1H à 4,82 ppm (D, J = 13Hz, $CH_2OCO$)- 1H à 4,33 ppm

(M, $\underline{CH}-NH_3^+)-$
$\quad | $
$\quad (CH_2)_2NH_3^+$

2H à 3,64 ppm (AB, $J_{AB}$ = 17Hz, $CH_2S$)- 2H à 2,90 ppm

(M, $CH-NH_3^+$ )-
$\quad | $
$\quad CH_2\underline{CH_2}NH_3^+$

2H à 2,15 ppm

(M, $CH-NH_3^+)-$
$\quad | $
$\quad \underline{CH_2}CH_2NH_3^+$

6H à 1,39 ppm (2S, $C(CH_3)_2$)

SPECTRE DE RMN N°20

1H à 10,80 ppm (S, OH)- 1H à 10,05 ppm (S, Ar NHCO)- 1H à 9,62 ppm (D, J = 9Hz, CONH)- 3H à 8,40 ppm (s. e., $NH_3^+$ )- 1H à 8,10 ppm (D, J = 8Hz, H aromatique para OH)- 3H à 7,85 ppm (s. e., $NH_3^+$ )- 2H à 7,45 ppm (M, H aromatiques)- 2H à 7,20 ppm (s. e., $NH_2$ thiazole)-1H à 6,66 ppm (S, H thiazole)- 1H à 5,76 ppm (D de D, $J_1$ = 9Hz $J_2$ = 4Hz, $H_7$)- 2H à 5,14 ppm (M, $H_6$ et $CH_2OCO$)- 1H à 4,95 ppm(D, J = 13Hz, $CH_2OCO$)- 1H à 4,35 ppm

(M, $\underline{CH}-NH_3^+)-$
$\quad | $
$\quad (CH_2)_2NH_3^+$

3H à 3,81 ppm (S, $NOCH_3$)- 2H à 3,60 ppm (M, $CH_2S$)- 2H à 2,88 ppm

(M, $CH-NH_3^+)-$
$\quad | $
$\quad CH_2\underline{CH_2}NH_3^+$

2H à 2,06 ppm (M, $CH-NH_3^+$)-
$|$
$\underline{CH_2}CH_2NH_3^+$

SPECTRE DE RMN N°21

1H à 10,75 ppm (s. e., $\underline{OH}$)- 1H à 10,01 ppm (S, Ar $\underline{NH}CO$)- 1H à 9,58 ppm (D, J = 9Hz, $CO\underline{NH}$)- 3H à 8,40 ppm (s. e., $NH_3^+$)- 1H à 8,09 ppm (D, J = 8Hz, H aromatique para OH)- 3H à 7,80 ppm (s. e., $NH_3^+$)- 2H à 7,46 ppm (M, H aromatiques)- 2H à 7,20 ppm (s. e., $NH_2$ thiazole)-1H à 6,71 ppm (S, H thiazole)- 1H à 5,79 ppm (D de D, $J_1$ = 9Hz $J_2$ = 4Hz, $H_7$)- 2H à 5,18 ppm (M, $H_6$ et $\underline{CH_2}OCO$)- 1H à 4,92 ppm (D, J = 13Hz, $\underline{CH_2}OCO$)- 1H à 4,30 ppm

(M, $\underline{CH}-NH_3^+$)-
$|$
$(CH_2)_3NH_3^+$

3H à 3,82 ppm (S, $NO\underline{CH_3}$)- 2H à 3,60 ppm (AB, $J_{AB}$ = 17Hz, $\underline{CH_2}S$)- 2H à 2,78 ppm

(M, $CH-NH_3^+$)- 2H à 1,80 ppm (M, $CH-NH_3^+$)- 2H à 1,62 ppm
$|$ $|$
$CH_2CH_2\underline{CH_2}NH_3^+$ $CH_2\underline{CH_2}CH_2NH_3^+$

(M, $CH-NH_3^+$)-
$|$
$\underline{CH_2}CH_2CH_2NH_3^+$

Les produits selon l'invention ont été étudiés en ce qui concerne leurs propriétés pharmacologiques.

L'action bactériostatique a été déterminée in vitro par la méthode des dilutions. L'étude a porté à la fois sur des souches à Gram positif et sur des souches à Gram négatif.

Les résultats, exprimés en concentrations minimales inhibitrices (CMI - µg/ml), pour divers produits selon l'invention sont rassemblés dans le tableau 3.

TABLEAU 3

| SOUCHES | PRODUITS N° SR | | | | | | | |
|---|---|---|---|---|---|---|---|---|
| | 43753 | 43852 | 43904 | 43853 | 44128 | 44219 | 44130 | 44218 |
| Staph. aureus Smith | 2 | 0,25 | 4 | 0,25 | 8 | 0,5 | 8 | 0,5 |
| Escherichia coli C1/Col E1 :: Tn3 | 0,12 | 0,06 | 0,25 | 0,06 | 0,12 | 0,12 | 0,25 | 0,25 |
| Escherichia coli SOL RL 90 | 2 | 0,25 | 2 | 0,25 | 2 | 0,12 | 2 | 0,5 |
| Klebsiella pneumoniae R30 | 1 | 4 | 1 | 4 | 1 | 4 | 1 | 8 |
| Proteus vulgaris GN 76/C-1 | 0,12 | 16 | 0,25 | 16 | 0,12 | 16 | 0,12 | 16 |
| Providencia 155 | 1 | 2 | 1 | 1 | 1 | 1 | 1 | 2 |
| Pseudomonas aeruginosa NCTC 8203 | 2 | 2 | 1 | 2 | 2 | 1 | 2 | 4 |

Ces résultats montrent que les produits selon l'invention présentent un spectre d'activité large et sont dotés d'une très bonne activité intrinsèque.

Par ailleurs le comportement pharmacocinétique des produits selon l'invention a été étudié chez le babouin après administration à la dose de 20 mg/kg par injection intramusculaire.

A partir de prélèvements sanguins effectués à divers temps après l'administration, on détermine la concentration plasmatique du produit étudié par dosage microbiologique.

On peut ainsi construire la courbe donnant la concentration plasmatique en fonction du temps et déterminer différents paramètres pharmacocinétiques du composé étudié :

- la demi vie d'élimination (tl/2 $\beta$) est calculée par la formule

$$\frac{\ln 2}{\beta}$$

où $\beta$ représente la pente d'élimination

- l'aire sous la courbe (AUC) est déterminée par la méthode des trapèzes.

Par ailleurs la liaison protéique est obtenue par comparaison de deux gammes étalon, l'une réalisée dans le plasma de babouin et l'autre dans le tampon phosphate (pH7, 0,03M).

Les concentrations plasmatiques obtenues avec divers produits de l'invention sont réunies dans le tableau 4. Elles sont exprimées en µg/ml.

A titre de comparaison, on a fait figurer dans ce tableau, les résultats obtenus avec 2 produits de l'art antérieur répondant respectivement aux formules :

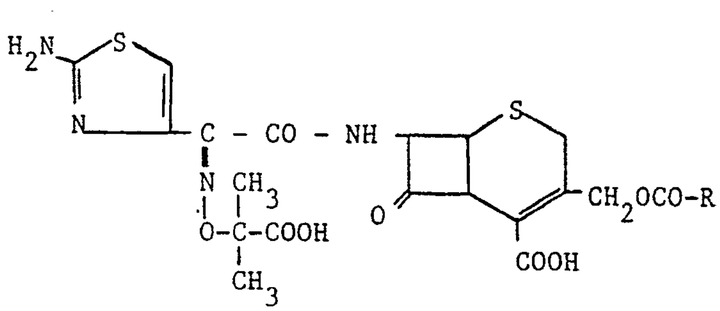 , $2CF_3COOH$

R = 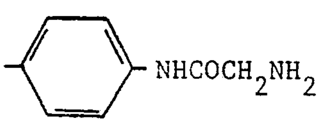 Composé A

R = 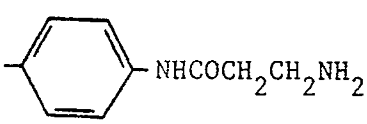 Composé B

TABLEAU 4

| TEMPS (minutes) | PRODUITS | | | | | |
|---|---|---|---|---|---|---|
| | SR 43753 | SR 43904 | SR 44128 | SR 44130 | A | B |
| 10 | 36,8 | 12,6 | 62,9 | 39,1 | 20,8 | 8,5 |
| 20 | 69,1 | ND | 115,3 | 60,9 | 36,8 | 16,4 |
| 30 | 95,2 | 52,4 | 134,8 | 81,9 | 44,4 | 16,9 |
| 45 | 128,6 | 73,0 | 176,6 | 104,7 | 59,3 | 17,6 |
| 60 | 139,2 | 76,1 | 187,9 | 121,6 | 60,0 | 20,7 |
| 90 | 146,0 | 94,1 | 187,8 | 143,4 | 55,5 | 24,1 |
| 120 | 151,1 | 76,1 | 194,6 | 141,6 | 53,3 | 20,7 |
| 180 | 126,1 | 59,7 | 168,8 | 137,8 | 40,0 | 17,6 |
| 240 | 120,9 | 57,3 | 141,9 | 134,8 | 36,2 | 12,5 |
| 300 | 112,6 | 53,4 | 123,0 | 109,1 | 27,2 | 8,3 |
| 330 | 97,6 | 39,9 | ND | 93,8 | 23,7 | 6,8 |
| 360 | 80,9 | 34,3 | 104,7 | 90,4 | 17,3 | 4,8 |

ND = non déterminé

Dans le tableau 5 ci-après, on a rassemblé pour les produits selon l'invention et les produits de comparaison les paramètres pharmacocinétiques déterminés d'après les mêmes expériences.

TABLEAU 5

| PARAMETRES | PRODUITS | | | | | |
|---|---|---|---|---|---|---|
| | SR 43753 | SR 43904 | SR 44128 | SR 44130 | A | B |
| Concentration plasmatique max (µg/ml) | 151,1 | 94,1 | 194,6 | 143,4 | 69,5 | 24,1 |
| T max (mn) * | 120 | 90 | 120 | 90 | 45 | 90 |
| Concentration plasmatique à 6h(µg/ml) | 80,9 | 34,3 | 104,5 | 90,4 | 17,3 | 4,8 |
| t1/2 β(mn) | NC | 265 | NC | NC | 195 | 99 |
| AUC 0-6h ($\mu g.ml^{-1}.mn$) | 42178 | 20859 | 53890 | 42403 | 17033 | 5276 |
| Excrétion urinaire (% dose,6h) | 18 | 17 | 72 | 37 | 23 | 18 |
| Liaison protéique % | 84 | 71 | 84 | 92 | 45 | 48 |

* T max est le temps auquel est atteint la concentration plasmatique maimum

NC : non calculable

Les résultats figurant dans les tableaux 4 et 5 montrent que les concentrations plasmatiques de produits de l'invention sont extrêmement élevées et prolongées.

Si l'on compare avec les produits de référence A et B les concentrations plasmatiques à 6 heures obtenues pour leurs homologues hydroxylés sont respectivement 4,6 et 7 fois plus élevées. De même si l'on considère l'aire sous la courbe l'augmentation est respectivement de 2,5 et 4 fois par rapport aux produits de référence.

Les composés selon l'invention présentent donc des paramètres pharmacocinétiques très intéressants qui peuvent permettre de diminuer de façon sensible la quantité de principe actif à utiliser et le nombre

d'administrations journalières nécessaires pour obtenir un effet thérapeutique donné.

Enfin la toxicité des produits selon l'invention est suffisamment faible pour permettre leur utilisation en thérapeutique.

Les produits de l'invention peuvent donc être employés comme antibiotiques en médecine humaine ou vétérinaire. Ils présentent un large spectre et peuvent être utilisés dans toutes les infections bactériennes à germes sensibles.

Les produits peuvent être administrés par voie générale (parentérale, orale, rectale) ou par voie topique.

Les compositions pharmaceutiques sont réalisées à partir des composés (I) sous une forme soluble obtenue par salification d'au moins une des fonctions acides ou des fonctions amines qui y sont présentes.

Les compositions pharmaceutiques contenant à titre d'ingrédient actif l'antibiotique selon l'invention en combinaison avec un véhicule pharmaceutiquement acceptable peuvent être solides ou liquides et se présenter, par exemple, sous forme de préparations injectables, de comprimés, gélules, granulés, pommades, crèmes, gels ou suppositoires. Elles contiendront, par exemple, de 50 à 1000 mg de principe actif. La posologie peut varier dans de larges proportions, en particulier suivant le type et la gravité de l'infection à traiter et suivant le mode d'administration.

Le plus souvent, chez l'adulte, par voie injectable, elle est comprise entre 0,250 g et 4g par jour.

A titre d'exemple de préparation galénique, on peut préparer une solution injectable contenant, pour chaque ampoule :

SR 43753 1 g
Eau pour préparation injectable 5 ml
Carbonate de sodium q.s. pour pH = 6,3

**Revendications**

**Revendications pour les Etats contractants suivants : AT, BE, CH, DE, FR, GB, IT, LI, LU, NL, SE**

**1.** Dérivés des céphalosporines répondant à la formule générale :

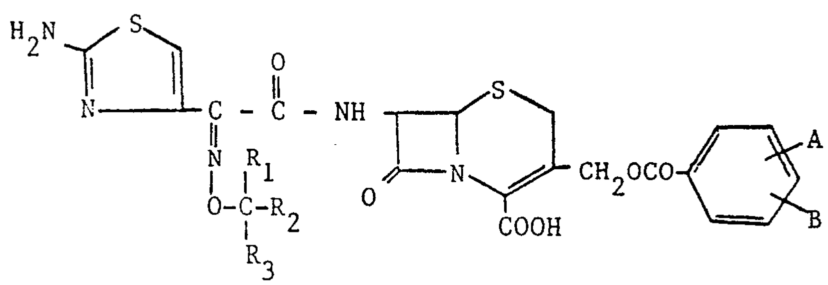

(I)

dans laquelle :

+ $R_1$, $R_2$ et $R_3$ désignent chacun un atome d'hydrogène, ou $R_1$ et $R_2$ désignent chacun un atome d'hydrogène ou un groupe méthyle et $R_3$ désigne un groupe carboxyle, ou encore $R_1$ et $R_2$ pris ensemble avec l'atome de carbone auquel ils sont liés forment un noyau cyclobutyle et $R_3$ désigne un groupe carboxyle,
+ A et B sont différents et occupent les positions méta et para du noyau benzénique ; l'un représente un groupe hydroxyle et l'autre désigne
- un groupe

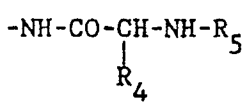

où $R_4$ représente l'hydrogène, un groupe méthyle, un groupe hydroxyméthyle, un groupe $(CH_2)_n$-$NH_2$ où n est un entier compris entre 1 et 4 et $R_5$ désigne l'hydrogène, ou lorsque $R_4$ représente l'hydrogène, $R_5$ peut aussi représenter un groupe alkyle inférieur comportant de 1 à 4 atomes de carbone ;
- un groupe $-NHCOCH_2CH_2NH_2$
- un groupe

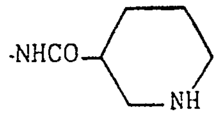

ainsi que les sels et les esters pharmaceutiquement acceptables des dits dérivés.

2. Dérivés des céphalosporines selon la revendication 1, de formule (I) dans laquelle le groupe hydroxyle est en position méta du noyau benzénique.

3. Dérivés des céphalosporines selon l'une des revendications 1 ou 2, de formule (I) dans laquelle l'oxime est sous la forme syn.

4. Dérivés des céphalosporines selon l'une quelconque des revendications 1 à 3, de formule (I) dans laquelle A représente OH en position méta et B désigne $-NH-CO-CH_2NH_2$ en position para.

5. Procédé de préparation des dérivés selon l'une quelconque des revendications 1 à 4, caractérisé en ce qu'il consiste :
   - à faire réagir en solution dans un solvant aprotique polaire sur un composé de formule

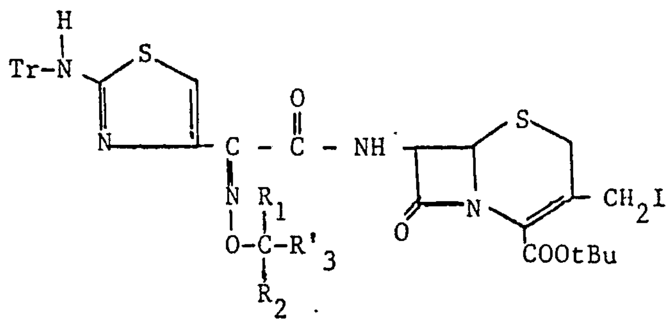

dans lequel $R_1$ et $R_2$ sont tels que définis à la revendication 1, $R'_3$ désigne l'hydrogène ou un groupe ester facilement labile et Tr représente un groupe protecteur de la fonction amine, un acide de formule

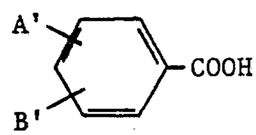 (ou l'un de ses dérivés réactifs)

dans laquelle A' et B' qui occupent les positions méta et para du cycle benzénique sont différents et représentent l'un un groupe hydroxyle, l'autre un groupe correspondant aux groupes

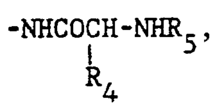, $-NHCOCH_2CH_2NH_2$, 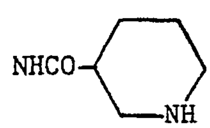

où $R_4$ et $R_5$ sont tels que définis dans la revendication 1 et dont le ou les groupes aminés ont été protégés par un groupe labile
   - à éliminer les groupes protecteurs présents sur les fonctions amines et carboxyliques par hydrolyse en milieu acide fort,
   - à isoler le composé (I) ainsi obtenu sous la forme d'un sel des groupements aminés présents dans la molécule,

- à transformer éventuellement ledit sel en un autre sel des groupements aminés ,
- à isoler, éventuellement, à partir d'un sel d'amine le composé I correspondant sous forme de base et à transformer éventuellement celle-ci selon des procédés connus en un sel ou un ester dérivant d'un ou des deux groupes carboxyliques présents dans la molécule.

**6.** Compositions pharmaceutiques contenant en tant que principe actif au moins un des composés de formule (I), selon l'une quelconque des revendications 1 à 4.

**7.** Compositions pharmaceutiques selon la revendication 6, sous forme d'unités de dosage, dans lesquelles le principe actif est mélangé à un excipient pharmaceutique.

**8.** Compositions pharmaceutiques selon l'une des revendications 6 ou 7, caractérisées en ce qu'elles contiennent de 50 à 1000 mg de principe actif.

**Revendications pour l'Etat contractant suivant : ES**

**1.** Procédé de préparation de dérivés des céphalosporines, répondant à la formule générale :

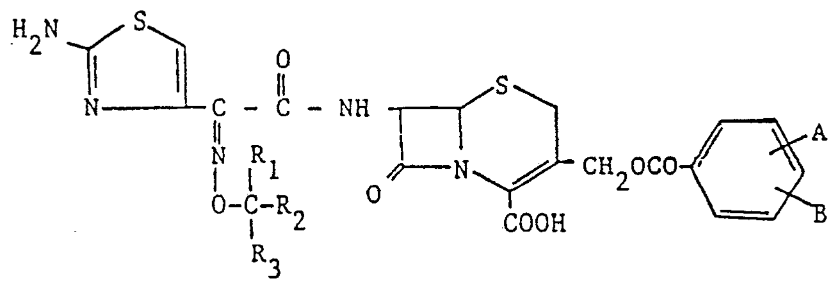

(I)

dans laquelle :

+ $R_1$, $R_2$ et $R_3$ désignent chacun un atome d'hydrogène, ou $R_1$ et $R_2$ désignent chacun un atome d'hydrogène ou un groupe méthyle et $R_3$ désigne un groupe carboxyle, ou encore $R_1$ et $R_2$ pris ensemble avec l'atome de carbone auquel ils sont liés forment un noyau cyclobutyle et $R_3$ désigne un groupe carboxyle,
+ A et B sont différents et occupent les positions méta et para du noyau benzénique ; l'un représente un groupe hydroxyle et l'autre désigne
- un groupe

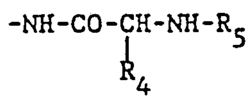

où $R_4$ représente l'hydrogène, un groupe méthyle, un groupe hydroxyméthyle, un groupe $(CH_2)_n$-$NH_2$ où n est un entier compris entre 1 et 4 et $R_5$ désigne l'hydrogène, ou lorsque $R_4$ représente l'hydrogène, $R_5$ peut aussi représenter un groupe alkyle inférieur comportant de 1 à 4 atomes de carbone ;
- un groupe -$NHCOCH_2CH_2NH_2$
- un groupe

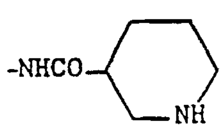

ainsi que des sels et des esters pharmaceutiquement acceptables des dits dérivés, caractérisé en ce qu'il consiste :

- à faire réagir en solution dans un solvant aprotique polaire sur un composé de formule

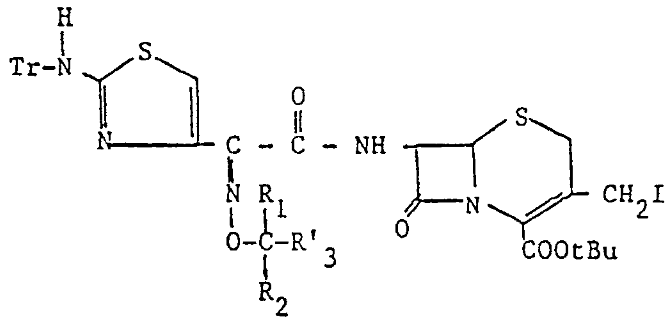

1

dans lequel $R_1$ et $R_2$ sont tels que définis ci-dessus $R'_3$ désigne l'hydrogène ou un groupe ester facilement labile et Tr représente un groupe protecteur de la fonction amine, un acide de formule

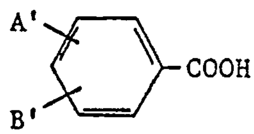 (ou l'un de ses dérivés réactifs) 2

dans laquelle A' et B' qui occupent les positions méta et para du cycle benzénique sont différents et représentent l'un un groupe hydroxyle, l'autre un groupe correspondant aux groupes

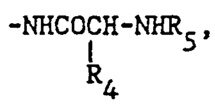, $-NHCOCH_2CH_2NH_2$, 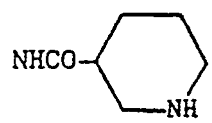

où $R_4$ et $R_5$ sont tels que définis ci-dessus,
et dont le ou les groupes aminés ont été protégés par un groupe labile

- à éliminer les groupes protecteurs présents sur les fonctions amines et carboxyliques par hydrolyse en milieu acide fort,
- à isoler le composé (I) ainsi obtenu sous la forme d'un sel des groupements aminés présents dans la molécule,
- à transformer éventuellement ledit sel en un autre sel des groupements aminés,
- à isoler, éventuellement, à partir d'un sel d'amine le composé I correspondant sous forme de base et à transformer éventuellement celle-ci selon des procédés connus en un sel ou un ester dérivant d'un ou des deux groupes carboxyliques présents dans la molécule.

2. Procédé selon la revendication 1, caractérisé en ce que, sur le composé de formule 1, on fait réagir un acide de formule

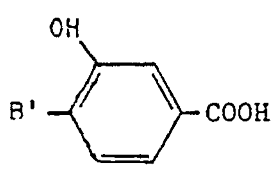 (ou l'un de ses dérivés réactifs) 2

dans laquelle B' est tel que défini à la revendication 1.

3. Procédé selon l'une des revendications 1 ou 2, caractérisé en ce que le groupe oxime du composé de formule 1 est sous la forme syn.

**4.** Procédé selon l'une quelconque des revendications 1 à 3, caractérisé en ce que, sur le composé de formule 1, on fait réagir un acide de formule :

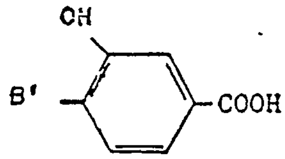

(ou l'un de ses dérivés réactifs) 2

dans laquelle B' correspond au groupe :
$-NH-CO-CH_2-NH_2$, dont les groupes aminés ont été protégés par un groupe labile.

**5.** Utilisation des dérivés de céphalosporines obtenus par le procédé selon l'une quelconque des revendications 1 à 4, pour la préparation de compositions pharmaceutiques.

**6.** Utilisation selon la revendication 5, pour la préparation de compositions pharmaceutiques, sous forme d'unités de dosage dans lesquelles lesdits dérivés de céphalosporines sont mélangés à un excipient pharmaceutique.

**7.** Utilisation selon l'une des revendications 5 ou 6 pour la préparation de compositions pharmaceutiques, contenant de 50 à 1000 mg desdits dérivés de céphalosporines.

**Revendications pour l'Etat contractant suivant : GR**

**1.** Dérivés des céphalosporines répondant à la formule générale :

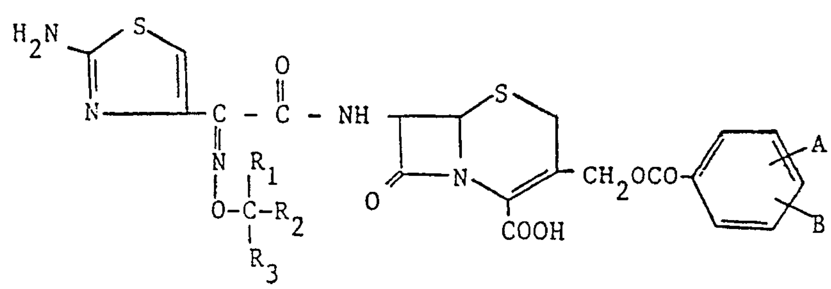

(I)

dans laquelle :

+ $R_1$, $R_2$ et $R_3$ désignent chacun un atome d'hydrogène, ou $R_1$ et $R_2$ désignent chacun un atome d'hydrogène ou un groupe méthyle et $R_3$ désigne un groupe carboxyle, ou encore $R_1$ et $R_2$ pris ensemble avec l'atome de carbone auquel ils sont liés forment un noyau cyclobutyle et $R_3$ désigne un groupe carboxyle,
+ A et B sont différents et occupent les positions méta et para du noyau benzénique ; l'un représente un groupe hydroxyle et l'autre désigne
- un groupe

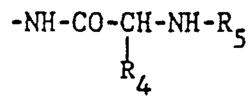

où $R_4$ représente l'hydrogène, un groupe méthyle, un groupe hydroxyméthyle, un groupe $(CH_2)_n-NH_2$ où n est un entier compris entre 1 et 4 et $R_5$ désigne l'hydrogène, ou lorsque $R_4$ représente l'hydrogène, $R_5$ peut aussi représenter un groupe alkyle inférieur comportant de 1 à 4 atomes de carbone ;
- un groupe $-NHCOCH_2CH_2NH_2$
- un groupe

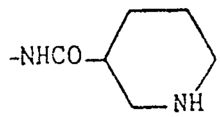

ainsi que les sols et les esters pharmaceutiquement acceptables des dits dérivés.

**2.** Dérivés des céphalosporines selon la revendication 1, de formule (1) dans laquelle le groupe hydroxyle est en position méta du noyau benzénique.

**3.** Dérivés des céphalosporines selon l'une des revendications 1 ou 2, de formule (I) dans laquelle l'oxime est sous la forme syn.

**4.** Dérivés des céphalosporines selon l'une quelconque des revendications 1 à 3, de formule (I) dans laquelle A représente OH en position méta et B désigne -MH-CO-$CH_2NH_2$ en position para.

**5.** Procédé de préparation des dérivés selon l'une quelconque des revendications 1 à 4, caractérisé en ce qu'il consiste :

- à faire réagir en solution dans un solvant aprotique polaire sur un composé de formule

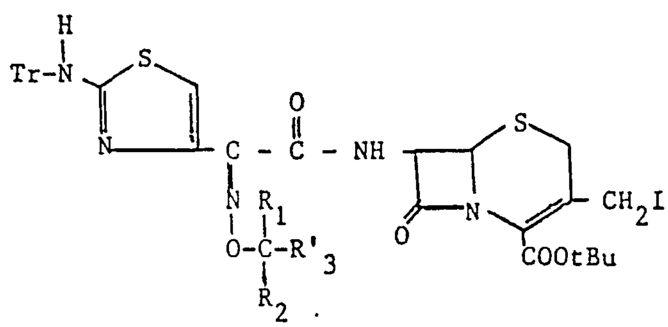

dans lequel $R_1$ et $R_2$ sont tels que définis à la revendication 1, $R'_3$ désigne l'hydrogène ou un groupe ester facilement labile et Tr représente un groupe protecteur de la fonction amine, un acide de formule

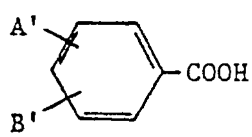 (ou l'un de ses dérivés réactifs)

dans laquelle A' et B' qui occupent les positions méta et para du cycle benzénique sont différents et représentent l'un un groupe hydroxyle, l'autre un groupe correspondant aux groupes

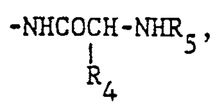, $-NHCOCH_2CH_2NH_2$, 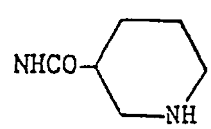

où $R_4$ et $R_5$ sont tels que définis dans la revendication 1 et dont le ou les groupes aminés ont été protégés par un groupe labile

- à éliminer les groupes protecteurs présents sur les fonctions amines et carboxyliques par hydrolyse en milieu acide fort,
- à isoler le composé (I) ainsi obtenu sous la forme d'un sel des groupements aminés présents dans la molécule,

- à transformer éventuellement ledit sel en un autre sel des groupements aminés,
- à isoler, éventuellement, à partir d'un sel d'amine le composé I correspondant sous forme de base et à transformer éventuellement celle-ci selon des procédés connus en un sel ou un ester dérivant d'un ou des deux groupes carboxyliques présents dans la molécule.

**6.** Utilisation des dérivés de céphalosporines selon l'une quelconque des revendications 1 à 4, pour la préparation de compositions pharmaceutiques.

**7.** Utilisation selon la revendication 6, pour la préparation de compositions pharmaceutiques, sous forme d'unités de dosage dans lesquelles lesdits dérivés de céphalosporines sont mélangés à un excipient pharmaceutique.

**8.** Utilisation selon l'une des revendications 6 ou 7 pour la préparation de compositions pharmaceutiques, contenant de 50 à 1000 mg desdits dérivés de céphalosporines.

**Claims**

**Claims for the following Contracting States : AT, BE, CH, DE, FR, GB, IT, LI, LU, NL, SE**

**1.** Cephalosporin derivatives corresponding to the general formula:

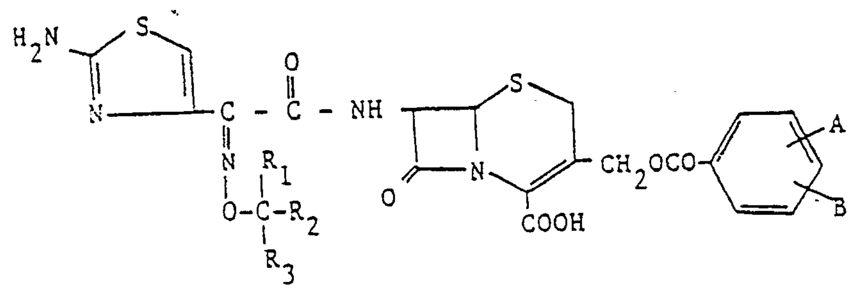

(I)

in which:

+ $R_1$, $R_2$ and $R_3$ each denote a hydrogen atom, or $R_1$ and $R_2$ each denote a hydrogen atom or a methyl group and $R_3$ denotes a carboxyl group, or $R_1$ and $R_2$, taken together with the carbon atom to which they are bonded, form a cyclobutyl ring and $R_3$ denotes a carboxyl group; and
+ A and B are different and occupy the meta and para positions of the benzene ring, one representing a hydroxyl group and the other denoting:
- a group

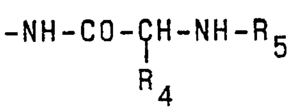

in which $R_4$ represents hydrogen, a methyl group, a hydroxymethyl group or a group$(CH_2)_n-NH_2$, in which n is an integer between 1 and 4, and $R_5$ denotes hydrogen, or when $R_4$ represents hydrogen, $R_5$ can also represent a lower alkyl group containing from 1 to 4 carbon atoms;
- a group $-NHCOCH_2CH_2NH_2$; or
- a group

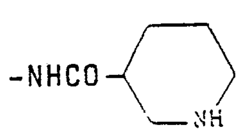

,

and also the pharmaceutically acceptable salts and esters of the said derivatives.

2. Cephalosporin derivatives according to claim 1, of the formula I, in which the hydroxyl group is in the meta position of the benzene ring.

3. Cephalosporin derivatives according to one of claims 1 or 2, of formula (I) in which the oxime is in the syn form.

4. Cephalosporin derivatives according to any one of claims 1 to 3, of formula (I) in which A represents OH in the meta position and B denotes $-NHCOCH_2NH_2$ in the para position.

5. Process for the preparation of the derivatives according to any one of claims 1 to 4, characterized in that it consists in:
   - reacting, in solution in a polar aprotic solvent, a compound of the formula:

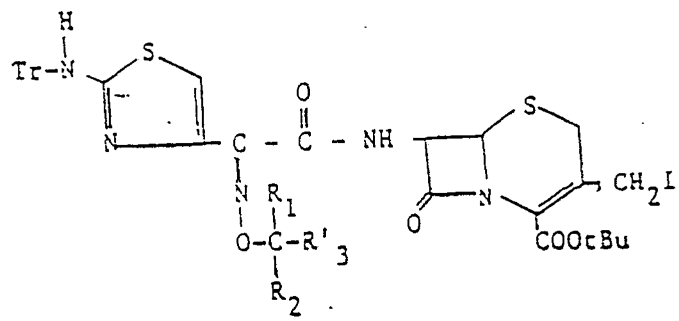

in which $R_1$ and $R_2$ are as defined in claim 1, $R'_3$ denotes hydrogen or a readily labile ester group and Tr represents a group protecting the amine function, with an acid of the formula:

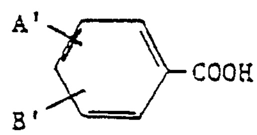 (or one of its reactive derivatives)

in which A' and B', which occupy the meta and para positions of the benzene ring, are different, one of them representing a hydroxyl group and the other representing a group corresponding to the groups:

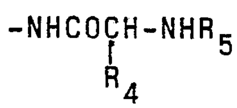, $-NHCOCH_2CH_2NH_2$, 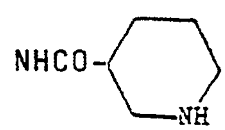

in which $R_4$ and $R_5$ are as defined in claim 1 and in which the amino group or groups have been protected by a labile group
   - removing the protecting groups present on the amine and carboxyl functions by hydrolysis in a strong acid medium;
   - isolating the resulting compound (I) in the form of a salt of the amino groups present in the molecule;
   - if appropriate, converting the said salt to another salt of the amino groups; and
   - if appropriate, isolating, from an amine salt, the corresponding compound I in the form of the base and, if appropriate, converting it by known processes, to a salt or ester derived from one or both of the carboxyl groups present in the molecule.

6. Pharmaceutical compositions containing as the active principle at least one of the compounds of formula (I) according to any one of claims 1 to 4.

7. Pharmaceutical composition according to claim 6, in the form of dosage units, in which the active principle is mixed with a pharmaceutical excipient.

8. Pharmaceutical compositions according to one of claims 6 or 7, characterized in that they contain 50 to 1000 mg of active principle.

**Claims for the following Contracting State : ES**

1. Process for the preparation of derivatives of cephalosporins, corresponding to general formula:

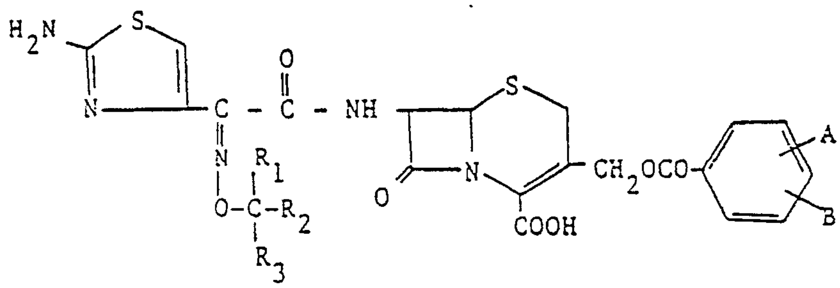

(I)

in which:

+ $R_1$, $R_2$ and $R_3$ each denote a hydrogen atom, or $R_1$ and $R_2$ each denote a hydrogen atom or a methyl group and $R_3$ denotes a carboxyl group, or $R_1$ and $R_2$, taken together with the carbon atom to which they are bonded, form a cyclobutyl ring and $R_3$ denotes a carboxyl group; and
+ A and B are different and occupy the meta and para positions of the benzene ring, one representing a hydroxyl group and the other denoting:
- a group

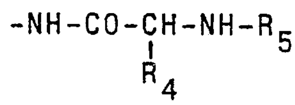

in which $R_4$ represents hydrogen, a methyl group, a hydroxymethyl group or a group $(CH_2)_n$-$NH_2$, in which n is an integer between 1 and 4, and $R_5$ denctes hydrogen, or when $R_4$ represents hydrogen, $R_5$ can also represent a lower alkyl group containing from 1 to 4 carbon atoms;
- a group -$NHCOCH_2CH_2NH_2$; or
- a group

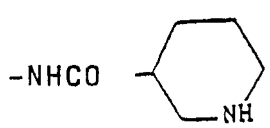

,

and also the pharmaceutically acceptable salts and esters of the said derivatives, characterized in that it consists in:
- reacting, in solution in a polar aprotic solvent, a compound of the formula:

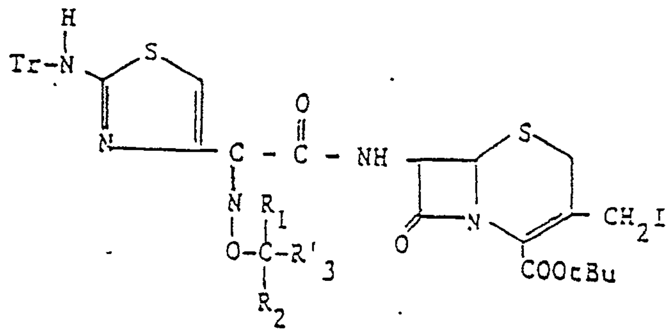

1

in which $R_1$ and $R_2$ are as defined above, $R'_3$ denotes hydrogen or a readily labile ester group and Tr represents a group protecting the amine function, with an acid of the formula:

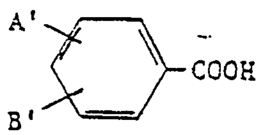

(or one of its reactive derivatives)

in which A' and B', which occupy the meta and para positions of the benzene ring, are different, one of them representing a hydroxyl group and the other representing a group corresponding to the groups:

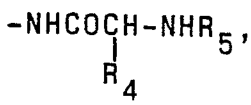, $-NHCOCH_2CH_2NH_2$ or NHCO- 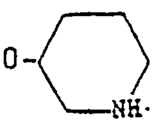

in which $R_4$ and $R_5$ are as defined above and in which the amino group or groups have been protected by a labile group;

- removing the protecting groups present on the amine and carboxyl functions by hydrolysis in a strong acid medium;
- isolating the resulting compound (I) in the form of a salt of the amino groups present in the molecule
- if appropriate, converting the said salt to another salt of the amino groups; and
- if appropriate, isolating, from an amine salt, the corresponding compound I in the form of the base and, if appropriate, converting it, by known processes, to a salt or ester derived from one or both of the carboxyl groups present in the molecule.

2. Process according to claim 1, characterized in that the compound of formula 1, is reacted with an acid of formula

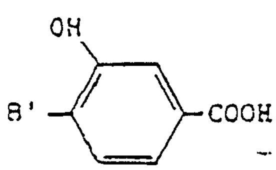

(or one of its reactive derivatives) 2

in which B' is as defined in claim 1.

3. Process according to one of claims 1 or 2, characterized in that the oxime of the compound of formula 1 is the syn form.

4. Process according to any one of claims 1 to 3, characterized in that the compound of formula 1 is reacted with an acid of formula:

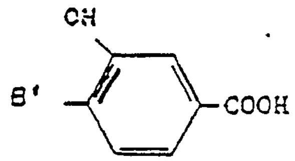 (or one of its reactive derivatives) 2

in which B' corresponds to the group $-NH-CO-CH_2-NH_2$, of which the amino groups have been protected by a labile group.

5. Use of the cephalosporin derivatives obtained with the process according to any one of claims 1 to 4, for the preparation of pharmaceutical compositions.

6. Use according to claim 5, for the preparation of pharmaceutical compositions, in the form of dosage units, in which said cephalosporin derivatives are mixed with a pharmaceutical excipient.

7. Use according to one of claims 5 or 6, for the preparation of pharmaceutical compositions containing 50 to 1000 mg of said cephalosporine derivatives.

**Claims for the following Contracting State : GR**

1. Cephalosporin derivatives corresponding to the general formula:

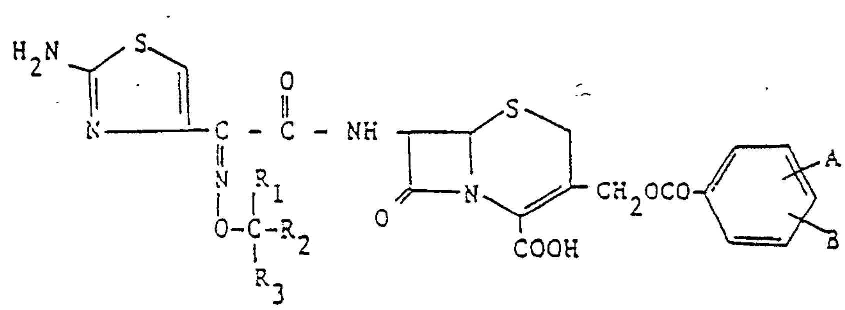 (I)

in which:

+ $R_1$, $R_2$ and $R_3$ each denote a hydrogen atom, or $R_1$ and $R_2$ each denote a hydrogen atom or a methyl group and $R_3$ denotes a carboxyl group, or $R_1$ and $R_2$, taken together with the carbon atom to which they are bonded, form a cyclobutyl ring and $R_3$ denotes a carboxyl group; and
+ A and B are different and occupy the meta and para positions of the benzene ring, one representing a hydroxyl group and the other denoting:
- a group

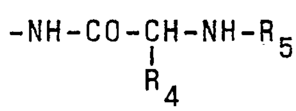

in which $R_4$ represents hydrogen, a methyl group, a hydroxymethyl group or a group$(CH_2)_n-NH_2$, in which n is an integer between 1 and 4, and $R_5$ denotes hydrogen, or when $R_4$ represents hydrogen, $R_5$ can also represent a lower alkyl group containing from 1 to 4 carbon atoms;
- a group $-NHCOCH_2CH_2NH_2$; or
- a group

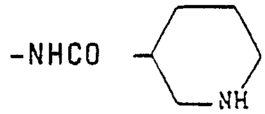 ,

and also the pharmaceutically acceptable salts and esters of the said derivatives.

2. Cephalosporin derivatives according to claim 1, of the formula I, in which the hydroxyl group is in the meta position of the benzene ring.

3. Cephalosporin derivatives according to one of claims 1 or 2, of formula (I) in which the oxime is in the syn form.

4. Cephalosporin derivatives according to any one of claims 1 to 3, of formula (I) in which A represents OH in the meta position and B denotes $-NHCOCH_2NH_2$ in the para position.

5. Process for the preparation of the derivatives according to any one of claims 1 to 4, characterized in that it consists in:
   - reacting, in solution in a polar aprotic solvent, a compound of the formula:

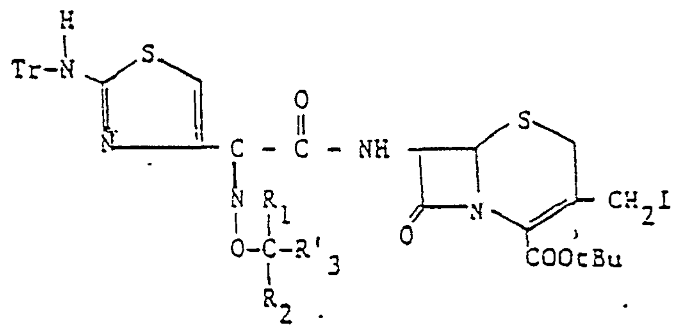

   in which $R_1$ and $R_2$ are as defined in claim 1, $R'_3$ denotes hydrogen or a readily labile ester group and Tr represents a group protecting the amine function, with an acid of the formula:

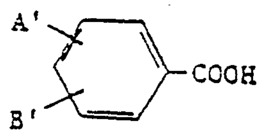 (or one of its reactive derivatives)

   in which A' and B' , which occupy the meta and para positions of the benzene ring, are different, one of them representing a hydroxyl group and the other representing a group corresponding to the groups:

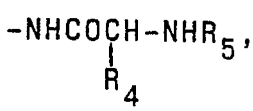 , $-NHCOCH_2CH_2NH_2$ or 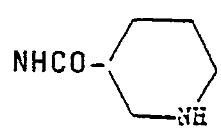

   in which $R_4$ and $R_5$ are as defined in claim 1 and in which the amino group or groups have been protected by a labile group;
   - removing the protecting groups present on the amine and carboxyl functions by hydrolysis in a strong acid medium;
   - isolating the resulting compound (I) in the form of a salt of the amino groups present in the

molecule;
- if appropriate, converting the said salt to another salt of the amino groups; and
- if appropriate, isolating, from an amine salt, the corresponding compound I in the form of the base and, if appropriate, converting it, by known processes, to a salt or ester derived from one or both of the carboxyl groups present in the molecule.

**6.** Use of the cephalosporin derivatives according to any one of claims 1 to 4 for the preparation of pharmaceutical compositions.

**7.** Use according to claim 6, for the preparation of pharmaceutical compositions in the form of dosage units, in which said cephalosporin derivatives are mixed with a pharmaceutical excipient.

**8.** Use according to any one of claim 6 or 7 for the preparation of pharmaceutical compositions; containing 50 to 1000 mg of said cephalosporin derivatives.

**Patentansprüche**
**Patentansprüche für folgende Vertragsstaaten : AT, BE, CH, DE, FR, GB, IT, LI, LU, NL, SE**

**1.** Cephalosporinderivate der allgemeinen Formel

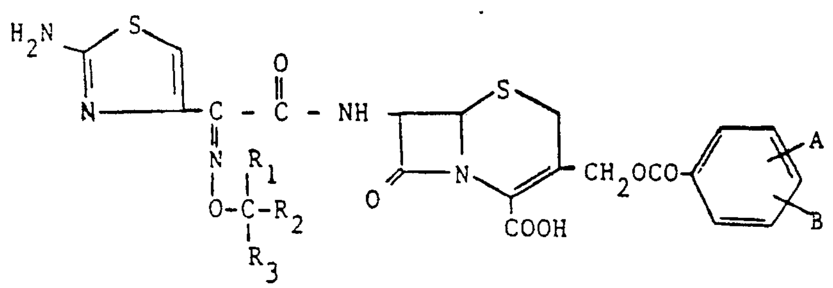

(I)

worin:
+ $R_1$, $R_2$ und $R_3$ jeweils ein Wasserstoffatom bezeichnen, oder $R_1$ und $R_2$ jeweils ein Wasserstoffatom oder eine Methylgruppe bezeichnen und $R_3$ eine Carboxylgruppe bedeutet, oder aber $R_1$ und $R_2$, zusammen mit dem Kohlenstoffatom, an das sie gebunden sind, einen Cyclobutylkern bilden und $R_3$ eine Carboxylgruppe bezeichnet,
+ A und B unterschiedlich sind und die meta- und para-Positionen des Benzolkerns einnehmen; eines repräsentiert eine Hydroxylgruppe und das andere bezeichnet
- eine Gruppe

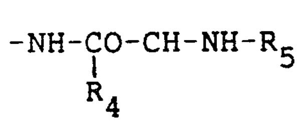

worin $R_4$ Wasserstoff, eine Methylgruppe, eine Hydroxymethylgruppe, eine Gruppe $(CH_2)_n$-$NH_2$, worin n für eine ganze Zahl zwischen 1 und 4 steht, darstellt und $R_5$ Wasserstoff bezeichnet, oder, wenn $R_4$ Wasserstoff darstellt, $R_5$ auch eine Niedrigalkylgruppe mit 1 bis 4 Kohlenstoffatomen darstellen kann;
- eine Gruppe $-NHCOCH_2CH_2NH_2$
- eine Gruppe

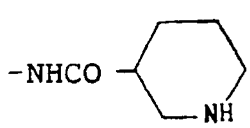

sowie die Salze und die pharmazeutisch akzeptablen Ester dieser Derivate.

2. Cephalosporinderivate nach Anspruch 1 der Formel (I), in welcher sich die Hydroxylgruppe in der meta-Position des Benzolkerns befindet.

3. Cephalosporinderivate nach einem der Ansprüche 1 oder 2 der Formel (I), in welcher das Oxim in syn-Form vorliegt.

4. Cephalosporinderivate nach einem der Ansprüche 1 bis 3 der Formel (I), in welcher A für OH in der meta-Position steht und B $-NH-CO-CH_{2NH2}$ in der para-Position bezeichnet.

5. Verfahren zur Herstellung der Derivate nach einem der Ansprüche 1 bis 4, dadurch gekennzeichnet, daß es darin besteht, daß
   - eine Verbindung der Formel

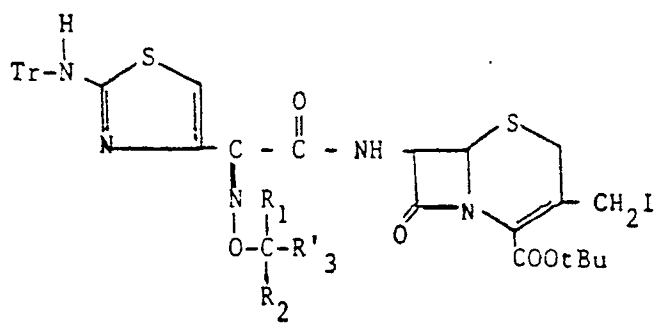

in welcher $R_1$ und $R_2$ wie in Anspruch 1 definiert sind, $R'_3$ Wasserstoff oder eine leicht labile Estergruppe bezeichnet und Tr eine Schutzgruppe der Aminfunktion darstellt, in Lösung in einem polaren aprotischen Lösungsmittel mit einer Säure der Formel

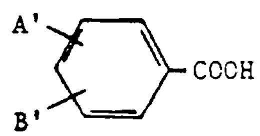 (oder einem ihrer reaktiven Derivate),

worin A' und B', die die meta- und para-Positionen des Benzolrings einnehmen, unterschiedlich sind und eines eine Hydroxylgruppe und das andere eine Gruppe entsprechend den Gruppen

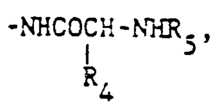, $-NHCOCH_2CH_2NH_2$, 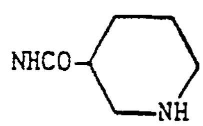,

worin $R_4$ und $R_5$ wie in Anspruch 1 definiert sind, darstellt, wobei die Aminogruppe(n) durch eine labile Gruppe geschützt ist/sind,
   - die an den Amin- und Carboxylfunktionen vorhandenen Schutzgruppen durch Hydrolyse in stark saurem Milieu entfernt werden,
   - die so in Form eines Salzes der im Molekül vorhandenen Aminogruppen erhaltene Verbindung (I) isoliert wird,
   - das Salz gegebenenfalls in ein anderes Salz der Aminogruppen übergeführt wird,
   - die entsprechende Verbindung I gegebenenfalls aus einem Aminsalz in Basenform isoliert und diese gegebenenfalls nach bekannten Verfahren in ein Salz oder einen Ester, abgeleitet von einer oder beiden im Molekül vorhandenen Carboxylgruppen, übergeführt wird.

6. Pharmazeutische Zusammensetzungen, die als Wirkstoff mindestens eine der Verbindungen der Formel (I) nach einem der Ansprüche 1 bis 4 enthalten.

7. Pharmazeutische Zusammensetzungen nach Anspruch 6 in Dosiseinheitsform, in denen der Wirkstoff mit einem pharmazeutischen Exzipienten vermischt ist.

8. Pharmazeutische Zusammensetzungen nach einem der Ansprüche 6 oder 7, dadurch gekennzeichnet, daß sie 50 bis 1000 mg Wirkstoff enthalten.

**Patentansprüche für folgenden Vertragsstaat : ES**

1. Verfahren zur Herstellung von Cephalosporinderivaten der allgemeinen Formel

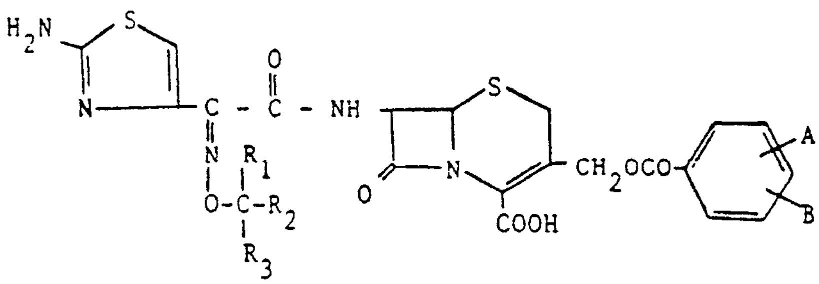

(I)

worin:

+ $R_1$, $R_2$ und $R_3$ jeweils ein Wasserstoffatom bezeichnen, oder $R_1$ und $R_2$ jeweils ein Wasserstoffatom oder eine Methylgruppe bezeichnen und $R_3$ eine Carboxylgruppe bedeutet, oder aber $R_1$ und $R_2$, zusammen mit dem Kohlenstoffatom, an das sie gebunden sind, einen Cyclobutylkern bilden und $R_3$ eine Carboxylgruppe bezeichnet,
+ A und B unterschiedlich sind und die meta- und para-Positionen des Benzolkerns einnehmen; eines repräsentiert eine Hydroxylgruppe und das andere bezeichnet
- eine Gruppe

$$-NH-\underset{\underset{R_4}{|}}{C}O-CH-NH-R_5$$

worin $R_4$ Wasserstoff, eine Methylgruppe, eine Hydroxymethylgruppe, eine Gruppe $(CH_2)_n$-$NH_2$, worin n für eine ganze Zahl zwischen 1 und 4 steht, darstellt und $R_5$ Wasserstoff bezeichnet, oder, wenn $R_4$ Wasserstoff darstellt, $R_5$ auch eine Niedrigalkylgruppe mit 1 bis 4 Kohlenstoffatomen darstellen kann;
- eine Gruppe $-NHCOCH_2CH_2NH_2$
- eine Gruppe

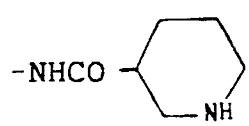

sowie der Salze und der pharmazeutisch akzeptablen Ester dieser Derivate, dadurch gekennzeichnet, daß es darin besteht, daß
- eine Verbindung der Formel

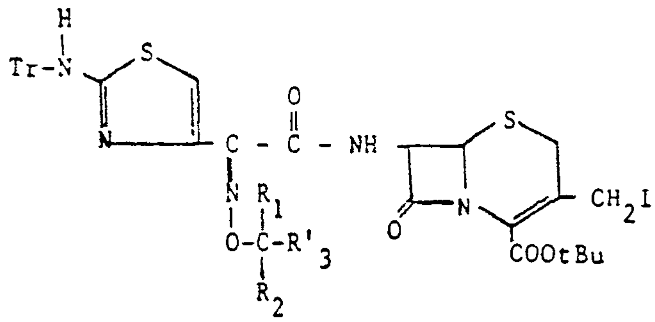

1

in welcher $R_1$ und $R_2$ wie oben definiert sind, $R'_3$ Wasserstoff oder eine leicht labile Estergruppe bezeichnet und Tr eine Schutzgruppe der Aminfunktion darstellt, in Lösung in einem polaren aprotischen Lösungsmittel mit einer Säure der Formel

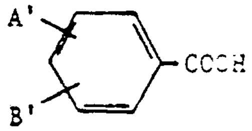 (oder einem ihrer reaktiven Derivate) 2

worin A' und B', die die meta- und para-Positionen des Benzolrings einnehmen, unterschiedlich sind und eines eine Hydroxylgruppe und das andere eine Gruppe entsprechend den Gruppen

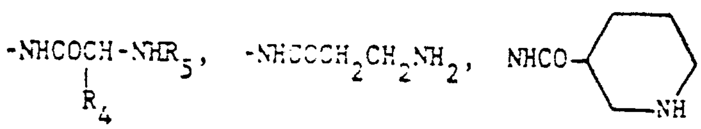 ,

worin $R_4$ und $R_5$ wie oben definiert sind, darstellt, wobei die Aminogruppe(n) durch eine labile Gruppe geschützt ist/sind,

- die an den Amin- und Carboxylfunktionen vorhandenen Schutzgruppen durch Hydrolyse in stark saurem Milieu entfernt werden,
- die so in Form eines Salzes der im Molekül vorhandenen Aminogruppen erhaltene Verbindung (I) isoliert wird,
- das Salz gegebenenfalls in ein anderes Salz der Aminogruppen übergeführt wird,
- die entsprechende Verbindung I gegebenenfalls aus einem Aminsalz in Basenform isoliert und diese gegebenenfalls nach bekannten Verfahren in ein Salz oder einen Ester, abgeleitet von einer oder beiden im Molekül vorhandenen Carboxylgruppen, übergeführt wird.

2. Verfahren nach Anspruch 1, dadurch gekennzeichnet, daß die Verbindung der Formel 1 mit einer Säure der Formel

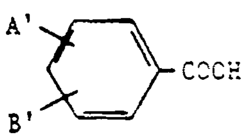 (oder einem ihrer reaktiven Derivate) 2

worin B' wie in Anspruch 1 definiert ist, zur Umsetzung gebracht wird.

3. Verfahren nach Anspruch 1 oder 2, dadurch gekennzeichnet, daß die Oximgruppe der Verbindung der Formel 1 in syn-Form vorliegt.

4. Verfahren nach einem der Ansprüche 1 bis 3, dadurch gekennzeichnet, daß die Verbindung der Formel 1 mit einer Säure der Formel

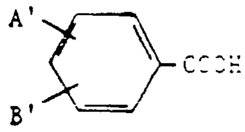 (oder einem ihrer reaktiven Derivate) 2

worin B' der Gruppe:
$-NH-CO-CH_2-NH_2$ entspricht, wobei die Aminogruppen durch eine labile Gruppe geschützt sind.

**5.** Verwendung der mittels des Verfahrens nach einem der Ansprüche 1 bis 4 erhaltenen Cephalosporinderivate zur Herstellung von pharmazeutischen Zusammensetzungen.

**6.** Verwendung nach Anspruch 5 zur Herstellung von pharmazeutischen Zusammensetzungen in Dosiseinheitsformen, in welchen die Cephalosporinderivate mit einem pharmazeutischen Exzipienten gemischt sind.

**7.** Verwendung nach einem der Ansprüche 5 oder 6 zur Herstellung von pharmazeutischen Zusammensetzungen, die 50 bis 1000 mg der Cephalosporinderivate enthalten.

**Patentansprüche für folgenden Vertragsstaat : GR**

**1.** Cephalosporinderivate der allgemeinen Formel

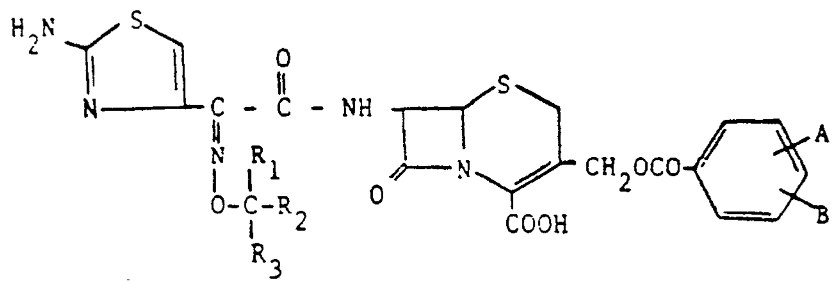 (I)

worin:

+ $R_1$, $R_2$ und $R_3$ jeweils ein Wasserstoffatom bezeichnen, oder $R_1$ und $R_2$ jeweils ein Wasserstoffatom oder eine Methylgruppe bezeichnen und $R_3$ eine Carboxylgruppe bedeutet, oder aber $R_1$ und $R_2$, zusammen mit dem Kohlenstoffatom, an das sie gebunden sind, einen Cyclobutylkern bilden und $R_3$ eine Carboxylgruppe bezeichnet,
+ A und B unterschiedlich sind und die meta- und para-Positionen des Benzolkerns einnehmen; eines repräsentiert eine Hydroxylgruppe und das andere bezeichnet
- eine Gruppe

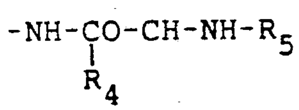

worin $R_4$ Wasserstoff, eine Methylgruppe, eine Hydroxymethylgruppe, eine Gruppe $(CH_2)_n-NH_2$, worin n für eine ganze Zahl zwischen 1 und 4 steht, darstellt und $R_5$ Wasserstoff bezeichnet, oder, wenn $R_4$ Wasserstoff darstellt, $R_5$ auch eine Niedrigalkylgruppe mit 1 bis 4 Kohlenstoffatomen darstellen kann;
- eine Gruppe $-NHCOCH_2CH_2NH_2$
- eine Gruppe

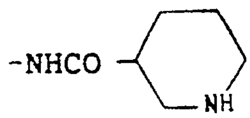

sowie die Salze und die pharmazeutisch akzeptablen Ester dieser Derivate.

2. Cephalosporinderivate nach Anspruch 1 der Formel (I), in welcher sich die Hydroxylgruppe in der meta-Position des Benzolkerns befindet.

3. Cephalosporinderivate nach einem der Ansprüche 1 oder 2 der Formel (I), in welcher das Oxim in syn-Form vorliegt.

4. Cephalosporinderivate nach einem der Ansprüche 1 bis 3 der Formel (I), in welcher A für OH in der meta-Position steht und B -NH-CO-$CH_{2NH2}$ in der para-Position bezeichnet.

5. Verfahren zur Herstellung der Derivate nach einem der Ansprüche 1 bis 4, dadurch gekennzeichnet, daß es darin besteht, daß
   - eine Verbindung der Formel

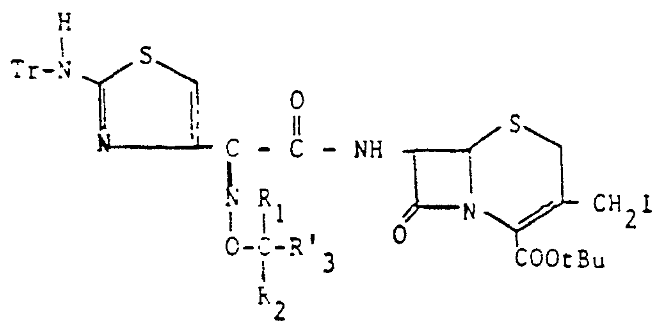

in welcher $R_1$ und $R_2$ wie in Anspruch 1 definiert sind, $R'_3$ Wasserstoff oder eine leicht labile Estergruppe bezeichnet und Tr eine Schutzgruppe der Aminfunktion darstellt, in Lösung in einem polaren aprotischen Lösungsmittel mit einer Säure der Formel

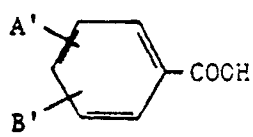 (oder einem ihrer reaktiven Derivate),

worin A' und B', die die meta- und para-Positionen des Benzolrings einnehmen, unterschiedlich sind und eines eine Hydroxylgruppe und das andere eine Gruppe entsprechend den Gruppen

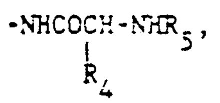, $-NHCOCH_2CH_2NH_2$, 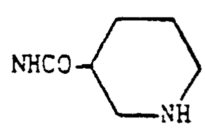 ,

worin $R_4$ und $R_5$ wie in Anspruch 1 definiert sind, darstellt, wobei die Aminogruppe(n) durch eine labile Gruppe geschützt ist/sind,
   - die an den Amin- und Carboxylfunktionen vorhandenen Schutzgruppen durch Hydrolyse in stark saurem Milieu entfernt werden,
   - die so in Form eines Salzes der im Molekül vorhandenen Aminogruppen erhaltene Verbindung (I) isoliert wird,

- das Salz gegebenenfalls in ein anderes Salz der Aminogruppen übergeführt wird,
- die entsprechende Verbindung I gegebenenfalls aus einem Aminsalz in Basenform isoliert und diese gegebenenfalls nach bekannten Verfahren in ein Salz oder einen Ester, abgeleitet von einer oder beiden im Molekül vorhandenen Carboxylgruppen, übergeführt wird.

**6.** Verwendung der Cephalosporinderivate nach einem der Ansprüche 1 bis 4 zur Herstellung von pharmazeutischen Zusammensetzungen.

**7.** Verwendung nach Anspruch 6 zur Herstellung von pharmazeutischen Zusammensetzungen in Dosiseinheitsformen, in welchen die Cephalosporinderivate mit einem pharmazeutischen Exzipienten gemischt sind.

**8.** Verwendung nach einem der Ansprüche 6 oder 7 zur Herstellung von pharmazeutischen Zusammensetzungen, die 50 bis 1000 mg der Cephalosporinderivate enthalten.